(19) Europäisches Patentamt
European Patent Office
Office européen des brevets

(11) EP 2 321 430 B1

(12) EUROPEAN PATENT SPECIFICATION

(45) Date of publication and mention of the grant of the patent:
**09.10.2013 Bulletin 2013/41**

(21) Application number: **09781439.6**

(22) Date of filing: **03.08.2009**

(51) Int Cl.:
*C12Q 1/68* (2006.01) *C12Q 1/70* (2006.01)
*G01N 33/52* (2006.01) *G01N 33/574* (2006.01)

(86) International application number:
**PCT/EP2009/060050**

(87) International publication number:
**WO 2010/015607 (11.02.2010 Gazette 2010/06)**

(54) **METHOD OF EVALUATING ORAL CANCER RISK IN HUMAN**

VERFAHREN ZUR BEWERTUNG DES ORALKARZINOMRISIKOS BEI MENSCHEN

PROCÉDÉ D'ÉVALUATION DU RISQUE D'UN CANCER DE LA BOUCHE CHEZ UN HUMAIN

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.08.2008 US 86019 P**

(43) Date of publication of application:
**18.05.2011 Bulletin 2011/20**

(73) Proprietor: **Institut Clinident Biopharma, S.A.S.**
**63360 Saint Beauzire (FR)**

(72) Inventor: **CHAUBRON, Franck**
**13770 Venelles (FR)**

(74) Representative: **Barbot, Willy et al**
**SIMODORO**
**Parc Cézanne II, Bât. G**
**290 Avenue Galilée**
**13857 Aix en Provence Cedex 3 (FR)**

(56) References cited:
**WO-A-2005/081867 WO-A-2006/020005**

- **LI Y ET AL: "Salivary transcriptome diagnostics for oral cancer detection" CLINICAL CANCER RESEARCH, THE AMERICAN ASSOCIATION FOR CANCER RESEARCH, US, vol. 10, no. 24, 15 December 2004 (2004-12-15), pages 8442-8450, XP002448846 ISSN: 1078-0432**
- **MAIE JOHN A R ST ET AL: "Interleukin 6 and interleukin 8 as potential biomarkers for oral cavity and oropharyngeal squamous cell carcinoma" ARCHIVES OF OTOLARYNGOLOGY HEAD AND NECK SURGERY, AMERICAN MEDICAL ASSOCIATION, US, vol. 130, no. 8, 1 August 2004 (2004-08-01), pages 929-935, XP002448845 ISSN: 0886-4470**
- **MAGER DL ET AL: "The salivary microbiota as a diagnostic indicator of oral cancer: A descriptive, non-randomized study of cancer-free and oral squamous cell carcinoma subjects" JOURNAL OF TRANSLATIONAL MEDICINE, BIOMED CENTRAL, LONDON, GB, vol. 3, no. 1, 7 July 2005 (2005-07-07), page 27, XP021009874 ISSN: 1479-5876 cited in the application**
- **THOMAS SHPITZER ET AL: "A comprehensive salivary analysis for oral cancer diagnosis" JOURNAL OF CANCER RESEARCH AND CLINICAL ONCOLOGY, SPRINGER, BERLIN, DE, vol. 133, no. 9, 4 May 2007 (2007-05-04), pages 613-617, XP019542570 ISSN: 1432-1335**
- **BAHAR GIDEON ET AL: "Salivary analysis in oral cancer patients - DNA and protein oxidation, reactive nitrogen species, and antioxidant profile" CANCER, vol. 109, no. 1, January 2007 (2007-01), pages 54-59, XP002550244 ISSN: 0008-543X**
- **PARK NOH JIN ET AL: "RNAprotect saliva: An optimal room- temperature stabilization reagent for the salivary transcriptome." CLINICAL CHEMISTRY DEC 2006, vol. 52, no. 12, December 2006 (2006-12), pages 2303-2304, XP002550245 ISSN: 0009-9147**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MACHADO ROBERTO F ET AL: "Detection of lung cancer by sensor array analyses of exhaled breath" AMERICAN JOURNAL OF RESPIRATORY AND CRITICAL CARE MEDICINE, vol. 171, no. 11, June 2005 (2005-06), pages 1286-1291, XP002565477 ISSN: 1073-449X
- MAZZONE PETER J: "Analysis of volatile organic compounds in the exhaled breath for the diagnosis of lung cancer." JOURNAL OF THORACIC ONCOLOGY : OFFICIAL PUBLICATION OF THE INTERNATIONAL ASSOCIATION FOR THE STUDY OF LUNG CANCER JUL 2008, vol. 3, no. 7, July 2008 (2008-07), pages 774-780, XP002565478 ISSN: 1556-1380
- DENG C ET AL: "Investigation of volatile biomarkers in lung cancer blood using solid-phase microextraction and capillary gas chromatography-mass spectrometry" JOURNAL OF CHROMATOGRAPHY B: BIOMEDICAL SCIENCES & APPLICATIONS, ELSEVIER, AMSTERDAM, NL, vol. 808, no. 2, 5 September 2004 (2004-09-05), pages 269-277, XP004521137 ISSN: 1570-0232
- HOMANN N ET AL: "Poor dental status increases acetaldehyde production from ethanol in saliva: a possible link to increased oral cancer risk among heavy drinkers" ORAL ONCOLOGY, ELSEVIER SCIENCE, OXFORD, GB, vol. 37, no. 2, 1 February 2001 (2001-02-01), pages 153-158, XP004249009 ISSN: 1368-8375
- CHIAPPIN ET AL: "Saliva specimen: A new laboratory tool for diagnostic and basic investigation" CLINICA CHIMICA ACTA, ELSEVIER BV, AMSTERDAM, NL, vol. 383, no. 1-2, 4 July 2007 (2007-07-04), pages 30-40, XP022142300 ISSN: 0009-8981
- MASHIR A ET AL: "Exhaled breath analysis: The new interface between medicine and engineering" ADVANCED POWDER TECHNOLOGY, VSP, UTRECHT, NL, vol. 20, no. 5, 1 September 2009 (2009-09-01), pages 420-425, XP026743255 ISSN: 0921-8831 [retrieved on 2009-06-14] cited in the application
- CLAUS DIRK ET AL: "Oral malodor, assessed by closed-loop, gas chromatography, and ion-trap technology", HRC JOURNAL OF HIGH RESOLUTION CHROMATOGRAPHY, vol. 20, no. 2, 1997, pages 94-98, XP55027372, ISSN: 0935-6304
- MANORI J SILVA ET AL: "Detection of phthalate metabolites in human saliva", ARCHIVES OF TOXICOLOGY, SPRINGER-VERLAG, BERLIN, DE, vol. 79, no. 11, 1 November 2005 (2005-11-01), pages 647-652, XP019325439, ISSN: 1432-0738, DOI: 10.1007/S00204-005-0674-4
- JONSSON B A G ET AL: "Determination of cortisol in human saliva using liquid chromatography-electrospray tandem mass spectrometry", JOURNAL OF CHROMATOGRAPHY B: BIOMEDICAL SCIENCES & APPLICATIONS, ELSEVIER, AMSTERDAM, NL, vol. 784, no. 1, 25 January 2003 (2003-01-25), pages 63-68, XP004399418, ISSN: 1570-0232, DOI: 10.1016/S1570-0232(02)00753-5
- LOCHNER A ET AL: "Gas chromatographic-mass spectrometric analysis of volatile constituents in saliva", JOURNAL OF CHROMATOGRAPHY B : BIOMEDICAL APPLICATIONS, ELSEVIER SCIENCE PUBLISHERS, NL, vol. 378, 1 January 1986 (1986-01-01), pages 267-282, XP026727830, ISSN: 0378-4347, DOI: 10.1016/S0378-4347(00) 80724-0 [retrieved on 1986-01-01]
- KOSTELC J G ET AL: "SALIVARY VOLATILES AS INDICATORS OF PERIODONTITIS", JOURNAL OF PERIODONTAL RESEARCH, BLACKWELL MUNKSGAARD, COPENHAGEN, DK, vol. 15, no. 2, 1 March 1980 (1980-03-01), pages 185-192, XP000564963, ISSN: 0022-3484, DOI: 10.1111/J. 1600-0765.1980.TB00273.X
- Nadia Fucci ET AL: "Simultaneous detection of some drugs of abuse in saliva samples by SPME technique.", Forensic Science International, vol. 134, no. 1, 1 June 2003 (2003-06-01), pages 40-45, XP55027382, ISSN: 0379-0738

SEQUENCE LISTING

<110> INSTITUT CLINIDENT CHAUBRON, Franck

<120> Methods of evaluating oral cancer risk in Human

<130> 354993D26501

<150> US61/086,019
<151> 2008-08-04

<160> 70

<170> PatentIn version 3.3

<210> 1
<211> 20
<212> DNA
<213> artificial sequence

<220>

<223> Primer forward

<400> 1
gtgggataac ctgccgaaag 20

<210> 2
<211> 25
<212> DNA
<213> artificial sequence

<220>
<223> Primer reverse

<400> 2
cccatccatt accgataaat cttta 25

<210> 3
<211> 21
<212> DNA
<213> artificial sequence

<220>
<223> Primer forward

<400> 3
ggcagactaa tacctgcata g 21

<210> 4
<211> 24
<212> DNA
<213> artificial sequence

<220>
<223> Primer reverse

<400> 4
atccattacc gataaacttt cttc 24

<210> 5
<211> 25
<212> DNA
<213> artificial sequence

<220>
<223> Primer reverse

<400> 5
catccccatc cattaccgat aaatc 25

<210> 6
<211> 21
<212> DNA
<213> artificial sequence

<220>
<223> Primer forward

<400> 6

taccgcataa gagtagatgt t 21

<210> 7
<211> 21
<212> DNA
<213> artificial sequence

<220>
<223> Primer forward

<400> 7
cgatagctaa taccgcataa g 21

<210> 8
<211> 21
<212> DNA
<213> artificial sequence

<220>
<223> Primer reverse

<400> 8
caggtccatc tggtagtgat g 21

<210> 9
<211> 23
<212> DNA
<213> artificial sequence

<220>
<223> Primer forward

<400> 9
ggatagcccg gagaaatttg gat 23

<210> 10
<211> 27
<212> DNA
<213> artificial sequence

<220>
<223> Primer reverse

<400> 10
cgtcatcaaa gtacacgtac tccttat 27

<210> 11
<211> 21
<212> DNA
<213> artificial sequence

<220>
<223> Primer forward

<400> 11
taccggatag gagcgtccac c 21

<210> 12

<211> 21
<212> DNA
<213> artificial sequence

<220>
<223> Primer reverse

<400> 12
taggccgcga gtccatccaa a 21

<210> 13
<211> 25
<212> DNA
<213> artificial sequence

<220>
<223> Primer forward

<400> 13
ttgcagatca tcaagaacac gtaga 25

<210> 14
<211> 27
<212> DNA
<213> artificial sequence

<220>
<223> Primer reverse

<400> 14
cagtagagat cagttgtctc tggttgc 27

<210> 15
<211> 19
<212> DNA
<213> artificial sequence

<220>
<223> Primer forward

<400> 15
agaggccagt gccattcgt 19

<210> 16
<211> 21
<212> DNA
<213> artificial sequence

<220>
<223> Primer reverse

<400> 16
gtttctctgc gtcgttggag t 21

<210> 17
<211> 18
<212> DNA
<213> artificial sequence

<220>
<223> Primer forward

<400> 17
cgtcttcccc tccatcgt 18

<210> 18
<211> 23
<212> DNA
<213> artificial sequence

<220>
<223> Primer reverse

<400> 18
agctcattgt agaaggtgtg gtg 23

<210> 19
<211> 21
<212> DNA
<213> artificial sequence

<220>
<223> Primer forward

<400> 19
aagacctgta cgccaacaca g 21

<210> 20
<211> 20
<212> DNA
<213> artificial sequence

<220>
<223> Primer reverse

<400> 20
cgtcatactc ctgcttgctg 20

<210> 21
<211> 22
<212> DNA
<213> artificial sequence

<220>
<223> Primer reverse

<400> 21
atactcctgc ttgctgatcc ac 22

<210> 22
<211> 21
<212> DNA
<213> artificial sequence

<220>
<223> Primer forward

<400> 22
ggatcagaaa ttctgaagaa t 21

<210> 23
<211> 21
<212> DNA
<213> artificial sequence

<220>
<223> Primer reverse

<400> 23
accctcttca ctggacagat c 21

<210> 24
<211> 21
<212> DNA
<213> artificial sequence

<220>
<223> Primer forward

<400> 24
ccagtgaaga gggttggaga c 21

<210> 25
<211> 22
<212> DNA
<213> artificial sequence

<220>
<223> Primer reverse

<400> 25
tggaggttgt catctacagc ag 22

<210> 26
<211> 20
<212> DNA
<213> artificial sequence

<220>
<223> Primer forward

<400> 26
ggcgctccgt gaaattagac 20

<210> 27
<211> 19
<212> DNA
<213> artificial sequence

<220>
<223> Primer reverse

<400> 27
cgctggaagg gaagtttgc 19

<210> 28
<211> 19
<212> DNA
<213> artificial sequence

<220>
<223> Primer forward

<400> 28
aaagcaccca ggaagcaac 19

<210> 29
<211> 22
<212> DNA
<213> artificial sequence

<220>
<223> Primer reverse

<400> 29
gcgaatcaga agttcagtgg ac 22

<210> 30
<211> 22
<212> DNA
<213> artificial sequence

<220>
<223> Primer forward

<400> 30
gagggttgtg gagaagtttt tg 22

<210> 31
<211> 22
<212> DNA
<213> artificial sequence

<220>
<223> Primer reverse

<400> 31
ctggcatctt cactgattct tg 22

<210> 32
<211> 30
<212> DNA
<213> artificial sequence

<220>
<223> Probe forward

<400> 32
gcatagtctt cgatgacggc atcagatttg 30

<210> 33
<211> 21
<212> DNA

<213> artificial sequence

<220>
<223> Probe forward

<400> 33
gatgttccat gacatttrct t 21

<210> 34
<211> 21
<212> DNA
<213> artificial sequence

<220>
<223> Probe forward

<400> 34
attggatggc atcatttgat a 21

<210> 35
<211> 18
<212> DNA
<213> artificial sequence

<220>
<223> Probe forward

<400> 35
catggtgggt gttggaaa 18

<210> 36
<211> 33
<212> DNA
<213> artificial sequence

<220>
<223> Probe forward

<400> 36
aatcatgcat ggagatacac ctacattgca tga 33

<210> 37
<211> 22
<212> DNA
<213> artificial sequence

<220>
<223> Probe forward

<400> 37
gctgcaaccg agcacgacag ga 22

<210> 38
<211> 18
<212> DNA
<213> artificial sequence

<220>

<223> Probe forward

<400> 38
caggcaccag ggcgtgat 18

<210> 39
<211> 16
<212> DNA
<213> artificial sequence

<220>
<223> Probe forward

<400> 39
cgagcacggc atcgtc 16

<210> 40
<211> 18
<212> DNA
<213> artificial sequence

<220>
<223> Probe forward

<400> 40
gcagatgtgg atcagcaa 18

<210> 41
<211> 22
<212> DNA
<213> artificial sequence

<220>
<223> Probe forward

<400> 41
aggatgcaga aggagatcac tg 22

<210> 42
<211> 21
<212> DNA
<213> artificial sequence

<220>
<223> Probe forward

<400> 42
taagccaggt tgcaatgagg t 21

<210> 43
<211> 21
<212> DNA
<213> artificial sequence

<220>
<223> Probe forward

<400> 43

gcactcctca ctcctctgtt g 21

<210> 44
<211> 26
<212> DNA
<213> artificial sequence

<220>
<223> Probe forward

<400> 44
tatcagaagt ccactgaact tctgat 26

<210> 45
<211> 29
<212> DNA
<213> artificial sequence

<220>
<223> Probe forward

<400> 45
atcagaagtc cactgaactt ctgatycgc 29

<210> 46
<211> 20
<212> DNA
<213> artificial sequence

<220>
<223> Probe forward

<400> 46
ggcgctccgt gaaattagac 20

<210> 47
<211> 19
<212> DNA
<213> artificial sequence

<220>
<223> Probe forward

<400> 47
ttcattctct gtggtatcc 19

<210> 48
<211> 17
<212> DNA
<213> artificial sequence

<220>
<223> Probe forward

<400> 48
ttcattctct gtggtat 17

<210> 49

<211> 30
<212> DNA
<213> artificial sequence

<220>
<223> Probe reverse

<400> 49
caaatctgat gccgtcatcg aagactatgc 30

<210> 50
<211> 21
<212> DNA
<213> artificial sequence

<220>
<223> Probe reverse

<400> 50
aagyaaatgt catggaacat c 21

<210> 51
<211> 21
<212> DNA
<213> artificial sequence

<220>
<223> Probe reverse

<400> 51
aagyaaatgt catggaacat c 21

<210> 52
<211> 21
<212> DNA
<213> artificial sequence

<220>
<223> Probe reverse

<400> 52
tatcaaatga tgccatccaa t 21

<210> 53
<211> 18
<212> DNA
<213> artificial sequence

<220>
<223> Probe reverse

<400> 53
tttccaacac ccaccatg 18

<210> 54
<211> 18
<212> DNA
<213> artificial sequence

<220>
<223> Probe reverse

<400> 54
atcacgccct ggtgcctg 18

<210> 55
<211> 16
<212> DNA
<213> artificial sequence

<220>
<223> Probe reverse

<400> 55
gacgatgccg tgctcg 16

<210> 56
<211> 22
<212> DNA
<213> artificial sequence

<220>
<223> Probe reverse

<400> 56
cagtgatctc cttctgcatc ct 22

<210> 57
<211> 21
<212> DNA
<213> artificial sequence

<220>
<223> Probe reverse

<400> 57
acctcattgc aacctggctt a 21

<210> 58
<211> 26
<212> DNA
<213> artificial sequence

<220>
<223> Probe reverse

<400> 58
atcagaagtt cagtggactt ctgata 26

<210> 59
<211> 20
<212> DNA
<213> artificial sequence

<220>
<223> Probe reverse

<400> 59
gtctaatttc acggagcgcc 20

<210> 60
<211> 17
<212> DNA
<213> artificial sequence

<220>
<223> Probe reverse

<400> 60
ataccacaga gaatgaa 17

<210> 61
<211> 1761
<212> DNA
<213> Homo sapiens

<220>
<221> misc_feature
<223> Human mRNA for beta-actin - NCBI X00351

<400> 61

```
ttgccgatcc gccgcccgtc cacacccgcc gccagctcac catggatgat gatatcgccg     60
cgctcgtcgt cgacaacggc tccggcatgt gcaaggccgg cttcgcgggc gacgatgccc    120
cccgggccgt cttcccctcc atcgtggggc gccccaggca ccagggcgtg atggtgggca    180
tgggtcagaa ggattcctat gtgggcgacg aggcccagag caagagaggc atcctcaccc    240
tgaagtaccc catcgagcac ggcatcgtca ccaactggga cgacatggag aaaatctggc    300
accacacctt ctacaatgag ctgcgtgtgg ctcccgagga gcaccccgtg ctgctgaccg    360
aggccccct gaaccccaag gccaaccgcg agaagatgac ccagatcatg tttgagacct    420
tcaacacccc agccatgtac gttgctatcc aggctgtgct atccctgtac gcctctggcc    480
gtaccactgg catcgtgatg gactccggtg acggggtcac ccacactgtg cccatctacg    540
aggggtatgc cctcccccat gccatcctgc gtctggacct ggctggccgg gacctgactg    600
actacctcat gaagatcctc accgagcgcg gctacagctt caccaccacg gccgagcggg    660
aaatcgtgcg tgacattaag gagaagctgt gctacgtcgc cctggacttc gagcaagaga    720
tggccacggc tgcttccagc tcctccctgg agaagagcta cgagctgcct gacggccagg    780
tcatcaccat tggcaatgag cggttccgct gccctgaggc actcttccag ccttccttcc    840
tgggcatgga gtcctgtggc atccacgaaa ctaccttcaa ctccatcatg aagtgtgacg    900
tggacatccg caaagacctg tacgccaaca cagtgctgtc tggcggcacc accatgtacc    960
ctggcattgc cgacaggatg cagaaggaga tcactgccct ggcacccagc acaatgaaga   1020
tcaagatcat tgctcctcct gagcgcaagt actccgtgtg gatcggcggc tccatcctgg   1080
cctcgctgtc caccttccag cagatgtgga tcagcaagca ggagtatgac gagtccggcc   1140
cctccatcgt ccaccgcaaa tgcttctagg cggactatga cttagttgcg ttacaccctt   1200
tcttgacaaa acctaacttg cgcagaaaac aagatgagat tggcatggct ttatttgttt   1260
tttttgtttt gttttggttt tttttttttt tttggcttga ctcaggattt aaaaactgga   1320
acggtgaagg tgacagcagt cggttggagc gagcatcccc caaagttcac aatgtggccg   1380
aggactttga ttgcacattg ttgttttttt aatagtcatt ccaaatatga gatgcattgt   1440
tacaggaagt cccttgccat cctaaaagcc accccacttc tctctaagga gaatggccca   1500
gtcctctccc aagtccacac aggggaggtg atagcattgc tttcgtgtaa attatgtaat   1560
gcaaaatttt tttaatcttc gccttaatac tttttttattt tgttttattt tgaatgatga   1620
gccttcgtgc cccccttcc cccttttgt cccccaactt gagatgtatg aaggcttttg   1680
gtctccctgg gagtgggtgg aggcagccag ggcttacctg tacactgact tgagaccagt   1740
tgaataaaag tgcacacctt a                                             1761
```

<210> 62
<211> 1060
<212> DNA
<213> Homo sapiens

<220>
<221> misc_feature
<223> Homo sapiens spermidine/spermine N1-acetyltransferase 1 (SAT1), mRNA NCBI NM_002970

<400> 62

```
cgcgggccga ctggtgttta tccgtcactc gccgaggttc cttgggtcat ggtgccagcc      60
tgactgagaa gaggacgctc ccgggagacg aatgaggaac cacctcctcc tactgttcaa     120
gtacaggggc ctggtccgca aagggaagaa aagcaaaaga cgaaaatggc taaattcgtg     180
atccgcccag ccactgccgc cgactgcagt gacatactgc ggctgatcaa ggagctggct     240
aaatatgaat acatggaaga acaagtaatc ttaactgaaa aagatctgct agaagatggt     300
tttggagagc acccctttta ccactgcctg gttgcagaag tgccgaaaga gcactggact     360
ccggaaggac acagcattgt tggttttgcc atgtactatt ttacctatga cccgtggatt     420
ggcaagttat tgtatcttga ggacttcttc gtgatgagtg attatagagg ctttggcata     480
ggatcagaaa ttctgaagaa tctaagccag gttgcaatga ggtgtcgctg cagcagcatg     540
cacttcttgg tagcagaatg gaatgaacca tccatcaact tctataaaag aagaggtgct     600
tctgatctgt ccagtgaaga gggttggaga ctgttcaaga tcgacaagga gtacttgcta     660
aaaatggcaa cagaggagtg aggagtgctg ctgtagatga caacctccat tctattttag     720
aataaattcc caacttctct tgctttctat gctgtttgta gtgaaataat agaatgagca     780
cccattccaa agctttatta ccagtggcgt tgttgcatgt ttgaaatgag gtctgtttaa     840
agtggcaatc tcagatgcag tttggagagt cagatctttc tccttgaata tctttcgata     900
aacaacaagg tggtgtgatc ttaatatatt tgaaaaaaac ttcattctcg tgagtcattt     960
aaatgtgtac aatgtacaca ctggtactta gagtttctgt ttgattcttt tttaataaac    1020

tactctttga tttaaaaaaa aaaaaaaaaa aaaaaaaaaa                         1060
```

<210> 63
<211> 1117
<212> DNA
<213> Homo sapiens

<220>
<221> misc_feature
<223> Homo sapiens H3 histone, family 3A (H3F3A), mRNA NCBI NM_002107

<400> 63

```
caattgtgtt cgcagccgcc gccgcgccgc cgtcgctctc caacgccagc gccgcctctc      60
gctcgccgag ctccagccga aggagaaggg gggtaagtaa ggaggtctct gtaccatggc     120
tcgtacaaag cagactgccc gcaaatcgac cggtggtaaa gcacccagga agcaactggc     180
tacaaaagcc gctcgcaaga gtgcgccctc tactggaggg gtgaagaaac ctcatcgtta     240
caggcctggt actgtggcgc tccgtgaaat tagacgttat cagaagtcca ctgaacttct     300
gattcgcaaa cttcccttcc agcgtctggt gcgagaaatt gctcaggact ttaaaacaga     360
tctgcgcttc cagagcgcag ctatcggtgc tttgcaggag gcaagtgagg cctatctggt     420
tggccttttt gaagacacca acctgtgtgc tatccatgcc aaacgtgtaa caattatgcc     480
aaaagacatc cagctagcac gccgcatacg tggagaacgt gcttaagaat ccactatgat     540
gggaaacatt tcattctcaa aaaaaaaaaa aaaaatttct cttcttcctg ttattggtag     600
ttctgaacgt tagatatttt ttttccatgg ggtcaaaagg tacctaagta tatgattgcg     660
agtggaaaaa taggggacag aaatcaggta ttggcagttt ttccattttc atttgtgtgt     720
gaatttttaa tataaatgcg gagacgtaaa gcattaatgc aagttaaaat gtttcagtga     780
acaagtttca gcggttcaac tttataataa ttataaataa acctgttaaa tttttctgga     840
caatgccagc atttggattt ttttaaaaca agtaaatttc ttattgatgg caactaaatg     900
gtgtttgtag catttttatc atacagtaga ttccatccat tcactatact tttctaactg     960
agttgtccta catgcaagta catgttttta atgttgtctg tcttctgtgc tgttcctgta    1020
agtttgctat taaaatacat taaactataa aaaaaaaaaa aaaaaaaaaa aaaaaaaaaa    1080
aaaaaaaaaa aaaaaaaaaa aaaaaaaaaa aaaaaaa                             1117
```

<210> 64
<211> 1665
<212> DNA
<213> Homo sapiens

<220>
<221> misc_feature
<223> Homo sapiens interleukin 8 (IL8), mRNA NM_000584

<400> 64

```
ctccataagg cacaaacttt cagagacagc agagcacaca agcttctagg acaagagcca      60
ggaagaaacc accggaagga accatctcac tgtgtgtaaa catgacttcc aagctggccg     120
tggctctctt ggcagccttc ctgatttctg cagctctgtg tgaaggtgca gttttgccaa     180
ggagtgctaa agaacttaga tgtcagtgca taaagacata ctccaaacct ttccacccca     240
aatttatcaa agaactgaga gtgattgaga gtggaccaca ctgcgccaac acagaaatta     300
ttgtaaagct ttctgatgga agagagctct gtctggaccc caaggaaaac tgggtgcaga     360
gggttgtgga gaagtttttg aagagggctg agaattcata aaaaaattca ttctctgtgg     420
tatccaagaa tcagtgaaga tgccagtgaa acttcaagca aatctacttc aacacttcat     480
gtattgtgtg ggtctgttgt agggttgcca gatgcaatac aagattcctg gttaaatttg     540
aatttcagta aacaatgaat agtttttcat tgtaccatga aatatccaga acatacttat     600
atgtaaagta ttatttattt gaatctacaa aaaacaacaa ataattttta aatataagga     660
ttttcctaga tattgcacgg gagaatatac aaatagcaaa attgaggcca agggccaaga     720
gaatatccga actttaattt caggaattga atgggtttgc tagaatgtga tatttgaagc     780
atcacataaa aatgatggga caataaattt tgccataaag tcaaatttag ctggaaatcc     840
tggatttttt tctgttaaat ctggcaaccc tagtctgcta gccaggatcc acaagtcctt     900
gttccactgt gccttggttt ctcctttatt tctaagtgga aaaagtatta gccaccatct     960
tacctcacag tgatgttgtg aggacatgtg gaagcacttt aagtttttc atcataacat     1020
aaattatttt caagtgtaac ttattaacct atttattatt tatgtattta tttaagcatc    1080
aaatatttgt gcaagaattt ggaaaaatag aagatgaatc attgattgaa tagttataaa    1140
gatgttatag taaatttatt ttattttaga tattaaatga tgttttatta gataaatttc    1200
aatcagggtt tttagattaa acaaacaaac aattgggtac ccagttaaat tttcatttca    1260
gataaacaac aaataatttt ttagtataag tacattattg tttatctgaa attttaattg    1320
aactaacaat cctagtttga tactcccagt cttgtcattg ccagctgtgt tggtagtgct    1380
gtgttgaatt acggaataat gagttagaac tattaaaaca gccaaaactc cacagtcaat    1440
attagtaatt tcttgctggt tgaaacttgt ttattatgta caaatagatt cttataatat    1500
tatttaaatg actgcatttt taaatacaag gctttatatt tttaacttta agatgttttt    1560
atgtgctctc caaatttttt ttactgtttc tgattgtatg gaaatataaa agtaaatatg    1620
aaacatttaa aatataattt gttgtcaaag taaaaaaaaa aaaaa                    1665
```

<210> 65
<211> 366
<212> DNA
<213> Prevotella melaninogenica

<220>
<221> misc_feature
<223> Prevotella melaninogenica partial 16S rRNA gene NCBI AJ555137

<400> 65

```
ggctcaggat gattttctag ctacaggctt aacacatgca agtcgagggg aaacggcatt      60
gagtgcttgc actctttgga cgtcgaccgg cgcacgggtg agtaacgcgt atccaacctt     120
cccattactg tgggataacc tgccgaaagg cagactaata cctgcatagt cttcgatgac     180
ggcatcagat ttgaagtaaa gatttatcgg taatggatgg ggatgcgtct gattagcttg     240
ttggcggggt aacggcccac caaggctacg atcagtaggg gttctgagag gaaggtcccc     300
cacattggaa ctgagacacg gcccaaactc ctacgggagg cagcaatcga attcccgctt     360
gccaca                                                                366
```

<210> 66
<211> 1454
<212> DNA
<213> Streptococcus mitis

<220>
<221> misc_feature
<223> Streptococcus mitis partial 16S rRNA gene, strain B6 NCBI AJ617805

<400> 66

```
cgctggcggc gtgcctaata catgcaagta gaacgctgaa ggaggagctt gcttctctgg      60
atgagttgcg aacgggtgag taacgcgtag gtaacctgcc tggtagcggg ggataactat     120
tggaaacgat agctaatacc gcataagagt agatgttgca tgacatttgc ttaaaaggtg     180
caattgcatc actaccagat ggacctgcgt tgtattagct agttggtggg gtaacggctc     240
accaaggcga cgatacatag ccgacctgag agggtgatcg gccacactgg gactgagaca     300
cggcccagac tcctacggga ggcagcagta gggaatcttc ggcaatggac ggaagtctga     360
ccgagcaacg ccgcgtgagt gaagaaggtt ttcggatcgt aaagctctgt tgtaagagaa     420
gaacgagtgt gagagtggaa agttcacact gtgacggtat cttaccagaa agggacggct     480
aactacgtgc cagcagccgc ggtaatacgt aggtcccgag cgttgtccgg atttattggg     540
cgtaaagcga gcgcaggcgg ttagataagt ctgaagttaa aggctgtggc ttaaccatag     600
tacgctttgg aaactgttta acttgagtgc aagaggggag agtggaattc catgtgtagc     660
ggtgaaatgc gtagatatat ggaggaacac cggtggcgaa agcggctctc tggcttgtaa     720
```

ctgacgctga ggctcgaaag cgtggggagc aaacaggatt agataccctg gtagtccacg 780
ccgtaaacga tgagtgctag gtgttagacc ctttccgggg tttagtgccg cagctaacgc 840
attaagcact ccgcctgggg agtacgaccg caaggttgaa actcaaagga attgacgggg 900
gcccgcacaa gcggtggagc atgtggttta attcgaagca acgcgaagaa ccttaccagg 960
tcttgacatc cctctgaccg ctctagagat agagttttcc ttcgggacag aggtgacagg 1020
tggtgcatgg ttgtcgtcag ctcgtgtcgt gagatgttgg gttaagtccc gcaacgagcg 1080
caacccctat tgttagttgc catcatttag ttgggcactc tagcgagact gccggtaata 1140
aaccggagga aggtggggat gacgtcaaat catcatgccc cttatgacct gggctacaca 1200
cgtgctacaa tggctggtac aacgagtcgc aagccggtga cggcaagcta atctcttaaa 1260
gccagtctca gttcggattg taggctgcaa ctcgcctaca tgaagtcgga atcgctagta 1320
atcgcggatc agcacgccgc ggtgaatacg ttcccgggcc ttgtacacac cgcccgtcac 1380
accacgagag tttgtaacac ccgaagtcgg tgaggtaacc gtaaggagcc agccgcctaa 1440
ggtttgatag atga 1454

<210> 67
<211> 1480
<212> DNA
<213> Capnocytophaga gingivalis

<220>
<221> misc_feature
<223> Capnocytophaga gingivalis 16S ribosomal RNA gene NCBI AF543295

<400> 67

```
agagtttgat cctggctcag atgaacgcta gcggcaggcc taacacatgc aagtcgaggg      60
agaagccctt tcgggggcag aaaccggcgc acgggtgcgt aacgcgtatg caacctacct     120
ttcacagggg gatagcccga agaaatttgg attaataccc cataatatta ttggatggca     180
tcatttgata attaaaactg cggtggtgaa agatgggcat gcgtcctatt agctagttgg     240
agtggtaacg gcacccaag gctacgatag gtaggggtcc tgagagggag atcccccaca      300
ctggtactga gacagggacc agaccctac gggaggcagc agtgaggaat attggtcaat      360
ggtcggaaga ctgaaccagc catgccgcgt gcaggaagaa tgccttatgg gttgtaaact     420
gcttttatat gggaagaata aggtgtacgt gtacattgat gacggtacca tatgaataag     480
catcggctaa ctccgtgcca gcagccgcgg taatacggag gatgcgagcg ttattcggaa     540
tcattgggtt taaagggtct gtaggcgggc tattaagtca ggggtgaaag gtttcagctt     600
aactgagaaa ttgcctttga tactggtagt cttgaatatc tgtgaagttc ttggaatgtg     660
tagtgtagcg gtgaaatgct tagatattac acagaacacc gattgcgaag gcaggggact     720
aacagacaat tgacgctgag agacgaaagc gtggggagcg aacaggaatt agatcccctg     780
gtagtccacg cctgtaaacg atggatacta gctgttgggc gcaggctgag ttggcttaag     840
cgaaagtgat aagtatccaa ccttggggaa gtacgcacgc aagtgtgaaa ctcaaaggaa     900
ttgacggggg cccgcacaag cggtggagca tgtggtttaa ttcgatgata cgcgaggaac     960
cttaccaagg tttaaatgga gactgacagg tgtagagata cgcccttctt cggacagttt    1020
tcaaggtgct gcatggttgt cgtcagctcg tgccgtgagg tgtcaggtta agtcctataa    1080
cgagcgcaac ccctattgtt agttaccagc aagtaaagtt ggggactcta gcaagactgc    1140
cggtgtaaac cgtgaggaag gtggggatga cgtcaaatca tcacggccct tacatcttgg    1200
gctacacacg tgctacaatg gtcgttacag agagcagcca ctgcgcgagc aggagcgaat    1260
ctataaagac ggtcacagtt cggatcggag tctgcaactc gactccgtga agctggaatc    1320
gctagtaatc ggatatcagc catgatccgg tgaatacgtt cccgggcctt gtacacaccg    1380
cccgtcaagc catggaagct gggagtacct gaagtcggtc accgcaagga gctgcctagg    1440
gtaaaaccag tgactggggc taagtcgtaa caaggtaacc                          1480
```

<210> 68
<211> 838
<212> DNA
<213> Micrococcus luteus

<220>
<221> misc_feature
<223> Micrococcus luteus partial 16S rRNA gene, isolate A24 NCBI AM285006

<400> 68

aggatgaacg ctggcggcgt gcttacacat gcaagtcgaa cgatgaagcc cagcttgctg 60
ggtggattag tggcgaacgg gtgagtaaca cgtgagtaac ctgcccttaa ctctgggata 120
agcctgggaa actgggtcta ataccggata ggagcgtcca ccgcatggtg ggtgttggaa 180
agatttatcg gttttggatg gactcgcggc ctatcagctt gttggtgagg taatggctca 240
ccaaggcgac gacgggtagc cggcctgaga gggtgaccgg ccacactggg actgagacac 300
ggcccagact cctacgggag gcagcagtgg ggaatattgc gcaatgggcg caagcctgat 360
gcagcgacgc cgcgtgaggg atgacggcct tcgggttgta aacctctttc agtagggaag 420
aagcgaaagt gacggtacct gcagaagaag caccggctaa ctacgtgcca gcagccgcgg 480
taatacgtag ggtgcgagcg ttatccggaa ttattgggcg taaagagctc gtaggcggtt 540
tgtcgcgtct gtcgtgaaag tccggggctt aaccccggat ctgcggtggg tacgggcaga 600
ctagagtgca gtaggggaga ctggaattcc tggtgtagcg gtggaatgcg cagatatcag 660
gaggaacacc gatggcgaag gcaggtctct gggctgtaac tgacgctgag gagcgaaagc 720
atggggagcg aacaggatta gataccctgg tagtccatgc cgtaaacgtt gggcactagg 780
tgtggggacc attccacggt ttccgcgcc gcagctaacg cattaagtgc cccgcccct 838

<210> 69
<211> 297
<212> DNA
<213> Human papillomavirus type 16

<220>
<221> misc_feature
<223> Human papillomavirus type 16 strain P209 E7 protein (E7) gene, complete cds NCBI EF422141

<400> 69

atgcatggag atacacctac attgcatgaa tatatgttag atttgcaacc agagacaact 60
gatctctact gttatgagca attaaatgac agctcagagg aggaggatga aatagatggt 120
ccagctggac aagcagaacc ggacagagcc cattacaata ttgtaacctt ttgttgcaag 180
tgtgactcta cgcttcggtt gtgcgtacaa agcacacacg tagacattcg tactttggaa 240
gacctgttaa tgggcacact aggaattgtg tgccccatct gttctcagaa accataa 297

<210> 70
<211> 477
<212> DNA
<213> Human papillomavirus type 18

<220>
<221> misc_feature
<223> Human papillomavirus type 18 strain P629 E6 protein (E6) gene, complete cds NCBI EF422111

<400> 70

```
atggcgcgct ttgaggatcc aacacggcga ccctacaagc tacctgatct gtgcacggaa    60
ctgaacactt cactgcaaga catagaaata acctgtgtat attgcaagac agtattggaa   120
cttacagagg tatttgaatt tgcattcaaa gatttatttg tagtgtatag agacagtata   180
ccgcatgctg catgccataa atgtatagat ttttattcta gaattagaga attaagacat   240
tattcagact ctgtgtatgg agacacatta gaaaaactaa ctaacactgg gttatacaat   300
ttattaataa ggtgcctgcg gtgccagaaa ccgttgaatc cagcagaaaa acttagacac   360
cttaatgaaa aacgacgatt ccacaaaata gctgggcact atagaggcca gtgccattcg   420
tgctgcaacc gagcacgaca ggagagactc caacgacgca gagaaacaca agtataa      477
```

## Description

The present application claims the priority of the US provisional patent application filed on August 4, 2008, under the serial number 61/086, 019.

The present invention relates to a method of providing a risk evaluation and diagnosis of human oral cancer by examining, in a saliva sample of a human subject, the presence of particular volatile biochemical organic compounds, a combination of which being indicative of an increased risk of oral cancer.

## BACKGROUND OF THE INVENTION

Cancers of the oral cavity accounted for 274,000 cases in 2002, with almost two-thirds of them in men. The world area with the highest incidence is Melanesia (31.5 per 100,000 in men and 20.2 per 100,000 in women). Rates in men are high in western Europe (11.3 per 100,000), southern Europe (9.2 per 100,000), south Asia (12.7 per 100,000), southern Africa (11.1 per 100,000), and Australia/New Zealand (10.2 per 100,000). In females, incidence of oral cancer is relatively high in southern Asia (8.3 per 100,000). These patterns reflect prevalence of specific risk factors, such as tobacco/alcohol, lack of dental and oral health and the chewing of betel quid in south central Asia and Melanesia. Moreover, for oral cavity cancer, the overall 5-year survival rates have not improved in the past several decades, remaining low at approximately 30-50% (Epstein, J.B. et al. 2002 J Can Dent Assoc 68: 617-621; Mao, L. et al. 2004 Cancer Cell 5: 311-316).

In addition, most of oral cancers are initially asymptomatic and are not diagnosed or treated until they reach an advanced stage. As of today, patients are questioned about associated risk to oral cancer (smoker, alcohol) followed by clinical inspection of oral cavity. Nonetheless, as indicated above early stages such as preneoplastic states and states of early tumor recurrence show no tissue damages that are visible by dentists or physicians.

Thus, there is a need for a method of assessing risk factor, for early diagnosis, and improved prognosis. In this regard, one problem is to provide a method that can be performed routinely and in the usual practice or laboratories.

The present invention disclosed a reliable and sensitive diagnostic method applied to the saliva of human subjects.

Saliva is a clear, slightly acidic fluid that contains a number of inorganic and organic constituents important to oral health. Whole saliva is a mix of secretions from major and minor salivary glands and gingival crevicular fluid, which contains sloughed host cells, bacteria and food debris.Therefore, saliva is not a passive "ultrafiltrate" of serum, but contains a distinctive composition of enzymes, hormones, antibodies, and other molecules (Rehak, N.N. et al. 2000 Clin Chem Lab Med 38:335-343; Wong DT, American Scientist, vol 96, 2008). For example, saliva contains a large number of proteins that aid in the protection of oral cavity tissues, including mucins, amylases, agglutinins, lisozymes, peroxidases, lactoferrin and secretory IgA. Whole saliva contains normal epithelial cells and leukocytes that can be pelleted, and from which one can easily recover genomic DNA and mRNA, potentially used to find genomic markers of several diseases. Indeed, most of the DNA or RNA extracted from crude saliva was found to be of viral or bacterial origin (Stamey, F.R. et al. 2003 J Virol Methods 108:189-193; Mercer, D.K. et al. 2001 FEMS Microbiol Lett 200:163-167) and of human extra or intracellular origin. Also, many groups have focused their study and diagnostic tests on the supernatant and thus cell-free phase of whole saliva, which contains many analytes such as free mRNA (Zimmermann BG et al, Oral Oncology 2008, 44, 425-429).

In the past 10 years, the use of saliva as been successfully applied in diagnostics (Streckfus, CF. & Bigler, L.R. 2002 Oral Dis 8:69-76). Diagnostic biomarkers in saliva have been identified for monitoring caries, periodontitis, salivary gland diseases, and systemic disorders, e.g., hepatitis and HIV (Lawrence H.P. et al, 2002 J Can Dent Assoc 68: 170-174). Also, oral bacteria have been reported to be elevated in oral and esophageal cancer lesions (Mager D.L. et al. J Transl Med. 2005; 3: 27, Hooper J.S et al., Journal of Clinical Microbiology, May 2006, P 1719-1725). The reason for these shifts in bacterial colonization of cancer lesions is unclear. Mechanistic studies of bacterial attachment provide some insights and research has repeatedly shown that oral bacteria demonstrate specific tropisms toward different biological surfaces in the oral cavity such as the teeth, mucosa, and other bacteria. There is less time in oral cavity, for a complex biofilm to develop on soft tissue surfaces; thus, a premium is placed on potent mechanisms of adhesion. The differences in bacterial tropisms for specific oral sites suggest that different intra-oral surfaces and bacterial species have different receptors and adhesion molecules that dictate the colonization of different oral surfaces. Certain glycoconjugates serve as receptors for specific bacteria and recent reports support the notion that shifts in the colonization of different cancer cells are associated with observed changes in cell surface receptors. Hence, Mager D.L. et al showed that the salivary microbiota in subjects with an oral squamous cell carcinoma (OSCC) lesion differs from that found in OSCC-free controls. Bacterial counts were determined for each species, averaged across subjects in the 2 subject groups, and significance of differences between groups determined using the Mann-Whitney test and adjusted for multiple comparisons; interestingly, it appeared that the bacteria strains *Capnocytophaga gingivalis, Prevotella melaninogenica, Streptococcus mitis* and *Micrococcus luteus* were particularly present in patients having OSCC and were therefore suggested to serve as diagnostic markers for oral cancer. However, as it is demonstrated in (ref), these particulate bacteria strains were poorly associated with oral cancer (a maximal sensitivity of 80%) (Mager D.L. et al). Also, it has been shown in Li et al (Journal of Applied Microbiology, 2004, 97, 1311-1318) that the presence in saliva of significant high numbers of specific alive bacteria (40 different strains have been identified in this study and more than 200 specific alive bacteria have been described in the oral cavity), could be associated to the biofilm formation, colonization of the oral cavity and lack of oral hygiene that are often associated to oral cancer development in developing countries. However, one can not predict from Li et al that the particulate strains *Capnocytophaga gingivalis, Prevotella melaninogenica, Streptococcus mitis* and *Micrococcus luteus* can serve as reliable diagnostic markers for human oral cancer. This is the reason why, to date, no reliable and very sensitive bacteria-based diagnostic test has ever been proposed to diagnose oral cancer in saliva.

It is interesting to note that the majority of species isolated were saccharolytic and acid tolerant and are known to produce short- chain organic acids from carbohydrates and consequently to lower the pH of their local environment. Raghunad N. et al . (J. Radiol. 2003; 76 . S11- S22) described the microenvironment of solid tumors as is typically hypoxic, with an acidic extracellular pH so it is not surprising that there might be a degree of selectivity in favor of acid tolerance. Other factor could be production of DNA oxidative damage generated by oral bacteria. Takeuchi T. et al. (2000, FEMS Microbiol Lett. Nov 1; 192 (l) : 133- 8), investigated the mechanism of the oxidative DNA damage induction by exposure to $O_2$ in *Prevotella melaninogenica,* a strict anaerobe found in oral cavity and some dental diseases. Results indicate that in *Prevotella melaninogenica,* exposure to $O_2$ generated and accumulated $O_2$ and $H_2O_2$, and that a crypto-OH radical generated through $H_2O_2$ was the active species in the 8OHdG induction, highly responsible for oxidative DNA damage in human cells (genotoxic effect), possible cause of mammalian cell tumorigenicity in the oral environment. Other bacterial pathogens have been described as possible source of oral tissus cancerisation.

James J. Closmann, also found that oral and oropharyngeal squamous cell carcinoma (OOSCC) have been linked to high- risk HPV strains, the same strains that cause cervical cancer in women. D'Souza et al. (N Engl J Med. 2007 May 10; 356 (19) : 1944- 56) concluded that oral HPV infection is strongly associated with oropharyngeal cancer among subjects with or without the established risk factors of tobacco and alcohol use. The degree of association increased with the number of vaginal- sex and oral- sex partners. Oropharyngeal cancer was associated with oral HPV type 16 (HPV- 16) infection; oral infection with any of 37 types of HPV and seropositivity for the HPV- 16 L1 capsid protein. HPV- 16 DNA was detected in 72% of 100 paraffin- embedded tumor specimens, and 64% of patients with cancer were seropositive for the HPV- 16 oncoprotein E6, E7, or both. HPV- 16 L1 seropositivity was highly associated with oropharyngeal cancer among subjects with a history of heavy tobacco and alcohol use. The association was similarly increased among subjects with oral HPV- 16. infection, regardless of their tobacco and alcohol use. Herrero R et al. (J Natl Cancer Inst. 2003 Dec 3; 95 (23) : 1772- 83) conducted a multicenter case- control study of cancer of the oral cavity and oropharynx in nine countries with 1670 case patients (1415 with cancer of the oral cavity and 255 with cancer of the oropharynx) and 1732 control subjects HPV DNA was detected by polymerase chain reaction (PCR) . Antibodies against HPV16 L1, E6, and E7 proteins in plasma were detected with enzyme- linked immunosorbent assays. Multivariable models were used for case- control and case- case comparisons. HPV DNA was detected in biopsy specimens of 3.9% of 766 cancers of the oral cavity with valid PCR results and 18.3% of 142 cancers of the oropharynx with valid PCR results. HPV16 DNA was found in 94.7% of HPV DNA- positive case patients. Consequently, it was suggested that antibodies against HPV16 L1 were associated with a high risk for cancers of the oral cavity and the oropharynx. Antibodies against HPV16 E6 or E7 were also associated with risk for cancers of the oral cavity and the oropharynx. HPV virus is even more of an indicator that patients should visit the dentist twice a year to identify anomalies early. Moreover, Rosenquist K. et al. (Acta Otolaryngol. 2007 Sep ; 127 (9) : 980- 7), described that high- risk orally HPV-infected patients have a significantly higher risk of recurrence/ second primary tumors compared with high- risk HPV-negative patients. Therefore, diagnosis of HPV in saliva of human subjects appears to be linked to a higher risk of developing oral cancer but also for monitoring associated treatment (antivirus) effects. However, to date, no HPV- based diagnostic test has ever been proposed to diagnose oral cancer in saliva.

On another hand, genetic aberrations of cancer cell lead to altered gene expression patterns (mRNA expression), which can be identified long before the resulting cancer phenotypes are manifested. Changes that arise exclusively or preferentially in cancer, compared with normal tissue of same origin, can be used as molecular biomarkers (Sidransky, D. 2002 Nat Rev Cancer 2: 210- 219, 2002) . Accurately identified, biomarkers may provide new avenues and constitute major targets for cancer early detection and cancer risk assessment. A variety of nucleic acid- based biomarkers have been demonstrated as novel and powerful tools for the detection of cancers (Hollstein, M. et al. 1991 Science 253: 49-53; Liu, T. et al. 2000 Genes Chromosomes Cancer 27: 17- 25; Groden, J. et al. 1991 Cell 66: 589- 600) . These nucleic-acid based biomarkers are mostly studied in the cell- free phase of whole saliva samples, i.e. in filtrated or centrifugated saliva samples (Zimmermann BG et al, Oral Oncology 2008, 44, 425- 429) . However, there are only a limited number of reports demonstrating tumor cell DNA heterogeneity in saliva of oral cancer patients (Liao P.H. et al. 2000 Oral Oncol 36: 272- 276; El- Naggar, A.K. et al. 2001 JMolDiagn 3: 164- 170) . Serum circulating human mRNA have been described for oral cancer detection (Yang Li et al, 2006, Journal of Clinical Oncology vol 24 number 11, 1754- 1760) . Using microarrays, profiling human salivary transcriptome (mRNA) have been realized on healthy controls and cancer patients (Hu S. and al. 2006, J Dent Res. Dec; 85 (12) : 1129- 33) . The different gene expression patterns were analyzed by combining a T test comparison and a fold- change analysis on 10 matched cancer patients and controls. The predictive power of these salivary mRNA biomarkers was analyzed by receiver operating characteristic curve and classification models. This microarray analysis showed that there are 1, 679 genes exhibiting significantly different expression level in saliva between cancer patients and controls, among which 7 cancer- related the mRNA of 1L8, IL1B, DUSPI, HA3, OAZ1, S100P, SAT are found. Combinations of these biomarkers yielded sensitivity (91%) and specificity (91%) in distinguishing oral cancer from the controls (WO2006/020005 or WO2005/081867) .

Saliva is a mixture of secretions from multiple salivary glands, including the parotid, submandibular, sublingual and other minor glands lying beneath the oral mucosa. As mentioned before, human saliva harbors a wide spectrum of peptides and proteins that constitutes the human salivary proteome. What has been less studied is the presence of organic biochemical compounds in the saliva.

Biochemical organic compounds can be enzymes, hormones, inorganic ions, peptides, proteins, carbohydrates, vitamins, lipids, fatty acids and volatile compounds. They can be measured by many techniques and devices, either optical technologies (for example laser absorption spectroscopy, mid infra red absorption spectroscopy, laser magnetic resonance spectroscopy, proton transfer reaction mass spectrometry...) or non-optical technologies (gas chromatography, mass spectrometry, etc...) (Mashir A, Advanced Powder Technology, 2009).

Only one study has ever compared the biochemical organic content of a fraction of saliva from healthy or sick-patients (Volozhin et al. Stomatologiia (mosk), 2001; 80 (1) : 9- 12) . In this study, patients with chronic generalized periodontitis and patients with chronic generalized gingivitis and periodontitis have been tested with air from the oral cavity and liquid samples were collected by washing the oral cavity with sterile water. Chemical compounds of the air and the washed liquid were analyzed by chromato- mass- spectrometry, gas- adsorption and gas- liquid chromatography. The content of dimethyl sulphide, dimethyl disulphide increased in the oral air and such volatile short chain fatty acids (VSCFA) as butyrate, propionate, acetate rose, but their aldehydes (butyraldehyde, acrolein, acetaldehyde) decreased in oral fluid during periodontitis. It was also shown that volatile short- chain fatty acids (propionate, butyrate and acetate) of bacterial and tissue origin are important factors of pathogenesis of oral tissue inflammation (Volozhin et al. Stomatologiia (mosk), 2001; 80 (1) : 9- 12) . In this study, the organic compounds have been analyzed in air from the oral cavity and rincing liquid collected by washing the oral cavity with sterile water but not in the volatile fraction of the raw saliva.

BAHAR et al. ("Salivary analysis in oral cancer patients - DNA and protein oxidation, reactive nitrogen species, and antioxidant profile" CANCER, vol. 109, no. 1, January 2007, pages 54-59) discloses salivary analysis in oral cancer patients, and finds that the salivary composition is altered in OSCC patients. Salivary reactive oxygen and nitrogen species such as NO, $NO_2$, $NO_3$, are substantially higher in oral cancer patients.

Contrary to saliva, the presence of volatile organic molecules in exhaled breath has been well studied and was shown to contain a lot of biochemical organic compounds: in 1971, using gas-liquid partition chromatography analysis, Linus Pauling demonstrated the presence of 250 substances in exhaled breath (Pauling L. et al. Proc.Natl.Acad.Sci.USA 68 (1971) 2374- 2376). In 1990, the development of very sensitive modem mass spectrometry (MS) and gas chromatography mass spectrometry (GC-MS) instruments, gives identity to thousands of unique substances in human exhaled breath (Mashir A, Advanced Powder Technology, 2009). These substances include elemental gases like nitric oxide and carbon monoxide and a multitude of other volatile organic compounds. Furthermore, exhaled breath also carries aerosolized droplets collected as exhaled breath condensate that have nonvolatile compounds that can be captured by a variety of methods and analyzed for a wide range of biomarkers from metabolic end products to proteins. Breath analysis is now used to diagnose and monitor asthma, pulmonary hypertension, respiratory diseases, gastrointestinal diseases, critical illness, to check for transplant organ rejection, and to detect lung cancer, and breast cancer (Mashir A, Advanced Powder Technology, 2009; Chan H.P. et al, Lung Cancer, 2009). However, it is noteworthy that breath analysis has never been proposed to detect oral cancer in human subject.

Interestingly, it appeared that the biochemical organic molecular composition of saliva has never been compared between patients suffering from cancer, e.g. oral cancer, and healthy individuals. Moreover, the biochemical organic molecular composition of the volatile fraction of saliva has never been studied so far.

The volatile fraction corresponds to the evaporated part of the fluid fraction of saliva. This volatile fraction contains some organic compounds that are not found in the raw saliva sample without the evaporation process, even after its filtration.

Also, it is important to note that the molecular content of saliva is not comparable at all to the composition of exhaled breath, which is mostly a reflect of lung molecules (Song G, et al. Quantitative breath analysis of volatile organic compounds of lung cancer patients, Lung Cancer (2009) ) . Hence, the molecular composition of the volatile fraction of saliva can not be inferred from the data issued from the breath analysis. To date, no exhaustive analysis of the biochemical content of the volatile fraction of saliva has never been disclosed, *a fortiori* in the context of oral cancer.

One of the values of saliva is the ease of sampling and subject compliance for sample collection, which includes field applications as well as home collection. However, the study of the biochemical compounds present in saliva (either in fluid or in the volatile fraction) for clinical application appeared to be difficult since it is necessary to stabilize and maintain their integrity for at least several days at room temperature.

It has already been shown that the RNAprotect® Saliva reagent (RPS, Qiagen Inc, Valencia, CA) could stabilize RNA in samples at room temperature for up to 12 weeks (Park NJ, Clin Chem 2006; 52:2303-4). Also, Jiang J et al showed that it is possible to use the RPS for stabilization of DNA and proteins in saliva only when endogenous cells are previously removed by centrifugation or by filtration (Jiang J et al, Archives of Oral Biology, 2008).

As far as biochemical organic compounds are concerned, they are degraded by the microflora, food and dental care products at room temperature. Importantly, nobody has ever proposed a way to protect these sensitive compounds from the degradation occuring at room temperature. *A fortiori,* a buffer enabling the stabilization of both nucleic acids and biochemical organic compounds for several days at room temperature has never been described so far.

Therefore, it is still a major challenge to stabilize all the components of saliva, especially nucleic acids and organic compounds, without any filtration or centrifugation steps, for a long time at room temperature.

In this context, the present inventors show here for the first time that:

i) it is possible to stabilize the nucleic acids and organic compounds present in saliva during at least 10 days in an appropriate buffer,

ii) it is possible to detect a high risk of developing oral cancer by analyzing the level of only few particular biomarkers in the fluid fraction of a stabilized sample of saliva,

iii) it is possible to extract a volatile fraction from a sample of stabilized saliva, and to detect therein several organic volatile compounds in a significant amount,

iv) it is possible to detect a high risk of developing oral cancer by analyzing the level of only few particular biochemical compounds in said volatile fraction of stabilized saliva.

Importantly, it is to note that no test for predicting and/or diagnosing oral cancer using the volatile fraction of saliva has ever been proposed so far.

More importantly, the particular biochemical compounds hereafter identified have never been associated with cancer so far.

The present specification therefore discloses:

i) a method for stabilizing crude saliva samples of human subjects,

ii) a method for detecting a high risk of developing oral cancer, by analyzing, in a one-step reaction, the level of particular biomarkers in the fluid fraction of said stabilized saliva,

iii) a method for extracting the volatile fraction of stabilized saliva samples, and to analyze its content in biochemical organic compounds,

iv) a method for detecting a high risk of developing oral cancer, by analyzing the level of particular organic biochemical compounds in the volatile fraction of stabilized saliva.

Finally, specific combinations of biological parameters associated with high risk of oral cancer have been identified, highlighting that it is possible to obtain a reliable and sensitive prognosis / diagnosis test of oral cancer from a unique sample of stabilized saliva.

## FIGURE DESCRIPTION

Figure 1 shows an histogram representing the 2 groups of saliva samples after a Factorial Discriminant Analysis (FDA) on ratios data according to the factor « tumor/healthy » : 5 ratios allow to separate 98.077% of the samples in the 2 groups « tumor » and « reference group » (only 1 reference sample is classified in the group « tumor » and not in the reference group).

## DESCRIPTION

The present invention disclose a new reliable, sensitive and easy to handle diagnostic test of oral cancer in human subject.

The present invention relates to an *in vitro* method of diagnosing a predisposition to oral cancer in a human subject, the method comprising stabilizing a crude saliva sample from said human subject, and performing at least the following step:

Analyzing a volatile fraction corresponding to the evaporable part extracted from said stabilized saliva by detecting in said volatile fraction at least one biochemical organic compound;

wherein the detection of at least one biochemical organic compound is indicative of a risk or a predisposition to oral cancer.

The present invention enables to determine if a human subject is predisposed of developing an oral cancer or not.

When a human subject is "diagnosed to have a predisposition to oral cancer" or is found to be "predisposed to oral cancer", it means that he has a higher risk of developing an oral cancer than the mean healthy population in the future. In other words, he is thought to be predisposed for developping an oral cancer in the early or far future. In the context of the invention, a human subject is said to be "predisposed to develop oral cancer" when he has a risk superior 70%, preferably 80%, more preferably 90% and even more preferably 95% of developing oral cancer in a short or far future as compared to a normal healthy population. This cancer predisposition is generally linked to a genetic cause.

The present invention also enables to determine that a human subject is "not predisposed to develop an oral cancer". In this case, it means that he has a poor risk of developing oral cancer in the future. For example, it means that he has a risk of developing an oral cancer in the future lower than 10%, preferably lower than 5% as compared to the normal healthy population. It generally means that he has at least 90% of chance not to have oncogenic mutations in his genome.

The present invention also enables to diagnose an oral cancer in a human subject. As a matter of fact, the present invention disclosed an *in vitro* method of diagnosing an oral cancer in a human subject, comprising stabilizing a crude saliva sample from said human subject, and performing at least the following step:

Analyzing a volatile fraction corresponding to the evaporable part extracted from said stabilized saliva by detecting in said volatile fraction at least one biochemical organic compound;

wherein the detection of at least one biochemical organic compound is indicative of a risk of developing oral cancer.

Preferably, the method of the invention further comprises the step of:

* Analyzing a fluid fraction of said stabilized saliva by detecting specific DNA or RNA sequences of human, bacterial or viral origin in said fluidic fraction.

The method of the invention is thus dedicated to estimate a risk for a human subject of developing an oral cancer. This risk can be either a high risk of developing an oral cancer or a low risk of developing an oral cancer.

As used herein, when a human subject has a risk "of developing" an oral cancer, it means that he has a risk "to be developing" an oral cancer at the time of the collection of the saliva sample.

In the context of the invention, a human subject is said "to have a high risk of developing an oral cancer" when he has a risk at least superior to 60%, preferably to 70%, more preferably to 80% and even more preferably to 90% of developing an oral cancer. In other words, the human subject has a much higher probability to develop an oral cancer as compared to the normal population or to a human subject in which none of the organic compound is detected. In the context of the present invention, when a human subject has a risk superior to 97% to be developing an oral cancer, it is said that the human subject "is developing an oral cancer".

This oral cancer can be initiating or well-established. In one embodiment of the invention, the level of expression of particulate biochemical organic compounds can potentially indicate the grade of the oral cancer from which the human sbject is suffering.

The method of the invention also enables to determine if a human subject has a low risk to be developing an oral cancer. In the context of the invention, the human subject has a low risk of developing an oral cancer when he has a risk of developing an oral cancer lower than 10%, preferably lower than 5% as compared with the normal population. In other words, the human subject has a chance superior to 90%, preferably 95% to be healthy, at least as far as oral cancer is concerned. In the context of the invention, when a human subject has a risk inferior to 5% of being developing an oral cancer, it is said that the human subject is not developing an oral cancer at the time of the collection of the saliva sample.

In the context of the invention, the term "oral cancer" triggers the following cancers: cancer of the oral cavity, cancer of the oropharynx, oropharyngeal squamous cell carcinoma (OSCC), head and neck squamous cell carcinoma, preferably oral squamous cell carcinoma.

In the context of the invention, "collecting a crude saliva sample" is obtained by receiving, in a sterile device, a sample of the saliva that has been spitted by the human subject. A collecting reagent, for example a citrate buffer, may be added to the sample. This sample is then treated so as to stabilize it for later analysis, and to prepare it to nucleic acid and/or volatile fraction extraction.

It is one aspect of the present disclosure to provide a way to stabilize crude samples of saliva, so as to maintain high amounts of genetic markers and biochemical components as present in the initially spitted and collected saliva and to favour nucleic acid and/or biochemical volatile elements extraction. The present invention enables to maintain preferably at least 70%, more preferably 80% and even more preferably 90% of the amount of nucleic acids and biochemical organic components initially present in the spitted and collected saliva.

More precisely, the method provided enables to protect the raw components of the sample from degradation during at least 10 days at room temperature. This stabilization step is performed by adding to the crude sample a so-called "saliva preservation solution" comprising at least a preservation reagent, which is preferably a buffer comprising a salt capable of a) opening the membrane of bacteria and human cells, b) blocking nuclease activity, c) precipitating the nucleic acid and d) reducing the vapour tension of volatile compounds without allowing the degradation of said compounds. This salt is preferably a salt such as guanidinum thiocyanate, and/or ammonium sulfate and/or sodium azide, preferably sodium azide. This salt is employed preferably at a concentration range between 20mM and 6 M, and more preferably at 40mM.

Therefore a method for stabilizing the raw components of a crude saliva sample (such as nucleic acid and biochemical organic compounds) during at least 10 days at room temperature is disclosed, said method comprising adding to the crude saliva sample a salt such as guanidinum thiocyanate, and/or ammonium sulfate and/or sodium azide. The salt is preferably sodium azide and is present at a concentration of about 40mM.

Therefore, by "stabilized" saliva sample, it is meant to refer to samples in which nucleic acid species and organic volatile compounds have been preserved from degradation caused by the microflora, food and dental care products, during at least 10 days at room temperature.

In a first aspect, a method of diagnosing a risk or a predisposition to oral cancer in a human subject, simply by using a stabilized sample of its saliva and analyzing its fluid fraction by detecting specific DNA or RNA sequences of human, bacterial or viral origin, is provided.

As used herein, the "fluid fraction" (or "fluidic fraction") of a saliva sample is the liquid phase of the collected saliva, and contains cells as well as free nucleic acids and organic compounds.

In the method provided, and contrary to what has been described so far, there is no need to remove the cells from the crude saliva samples to obtain the fluid fraction. Therefore, the saliva fluid samples are preferably not centrifugated and not filtrated.

As used herein, the expression "DNA or RNA sequences" refer to total nucleic acid in the sample and comprises genomic DNA and total RNA including mRNA.

Preferably, said specific DNA or RNA sequences are chosen among:

i) human sequences selected from SSAT mRNA (SEQ ID No 62), H3F3A mRNA (SEQ ID No 63) and IL8 mRNA (SEQ ID No 64); and/or

ii) sequences of bacteria selected from *Capnocytophaga gingivalis* (ATCC 33624), *Prevotella melaninogenica* (ATCC 25845), *Streptococcus mitis* (ATCC 15914) and *Micrococcus luteus* (ATCC 53598D); and/or

iii) the viral sequences of human papillomavirus, preferably the human papillomavirus 16 (ATCC 45113) or the human papillomavirus 18 (ATCC 45152).

Preferably, specific DNA or RNA sequences (also called nucleic acid sequences) are detected by incubating said genomic DNA and total RNA with a thermostable enzyme with RNA- dependent Reverse Transcriptase activity and with DNA- dependent Polymerase activity.

More preferably, the combination of RT and PCR is performed in a single-tube reaction.

The term "detecting" as used herein is meant to refer to diagnosing, inferring, evaluating, monitoring, determining the amount, concentration, ratio, or other quantitative or qualitative assessment in samples, optionally compared to a control sample, of nucleic acid and volatile compounds.

In the context of the present disclosure, "detecting specific nucleic acid sequences" comprises comparing the expression level of the specific nucleic acid sequences to the mean expression level of said specific nucleic acid sequences in the normal population. A specific nucleic acid sequence is detected when the expression level of said specific nucleic acid sequences in the tested saliva sample is equivalent or superior to 2 fold the mean expression level of said specific nucleic acid sequences in the normal population.

The method provided may comprise comparing the level of expression of specific nucleic acid sequence in a saliva sample from a subject, with the normal expression level of said nucleic acid sequence in a control. A significantly higher level of expression of said gene in the saliva sample of a subject as compared to the normal expression level is an indication that the patient has or is predisposed to oral cancer. An "over-expression" of a specific nucleic acid refers to an expression level in a saliva sample that is greater than the standard error of the assay employed to assess expression, and is preferably at least 20% superior to the normal level of expression of said nucleic acid, preferably at least 50% superior to the normal level of expression of said nucleic acid, and most preferably at least 100% superior to the normal level of expression of said nucleic acid. The "normal" level of expression of a nucleic acid is the level of expression of said nucleic acid in a saliva sample of a subject not afflicted with cancer. Preferably, said normal level of expression is assessed in a control sample (*e.g.,* sample from a healthy subject, which is not afflicted by cancer) and preferably, the mean expression level of said nucleic acid in several control samples.

Also, the detection of specific nucleic acid sequences is based on the detection of at least 50, preferably 70, and even more preferably 100 successive nucleotides of the specific targeted nucleic acid sequence as registered in the official data bases, for example in the NCBI data base.

For nucleic acid sequences according to ii) as defined above, "detecting" comprises comparing the expression level to the expression level in the normal population or to mean expression level and wherein when expression level is equivalent or superior of the threshold/cutoff of $10^5$ CFU per ml of saliva, it is indicative of increased oral cancer.

In one embodiment of the present invention, the detection of at least two human mRNA sequences as specifically defined in i) and at least one bacterial sequence as specifically defined in ii) indicates that the human subject has a high risk of developing an oral cancer.

In a preferred embodiment of the present invention, the detection of at least the human mRNA of H3F3A (SEQ ID No 63) and the human mRNA of SSAT (SEQ ID No 62) in the saliva sample of a human subject indicates that said human subject has a high risk developing an oral cancer.

In a more preferred embodiment of the present invention, the detection of the human mRNA of H3F3A (SEQ ID No 63), the human mRNA of SSAT (SEQ ID No 62) and the bacterial genome of *Streptococcus mitis* (ATCC 15914) in the saliva sample of a human subject indicates that said human subject has a risk superior to 64% of developing an oral cancer, and has therefore a high risk of developing an oral cancer.

On the contrary, when the particulate DNA or RNA sequence as disclosed in the present invention are not detected in the saliva sample of a human subject, it means that said human subject has a low risk of developing an oral cancer.

As mentioned previously, one problem was to design a test detecting the various risk factors actually associated with oral cancer in one simple test reaction. Indeed, some important biological markers are of different origins, virus, bacteria, human, or from mRNA or DNA, which makes the detection difficult in one step reaction test.

In one embodiment of the present invention, the detection of nucleic acid sequences comprises incubating said genomic DNA and total RNA with a thermostable enzyme with RNA-dependent Reverse Transcriptase activity and with DNA-dependent Polymerase activity, allowing the combination of RT-PCR and PCR.

Preferably, the RT- PCR reaction is performed with the Tth DNA polymerase.

More preferably, the detection of nucleic acid sequences comprises incubating said genomic DNA and total RNA in the same tube with a thermostable enzyme with RNA-dependent Reverse Transcriptase activity and with DNA-dependent Polymerase activity, allowing the combination of RT-PCR and PCR in a single-tube reaction. Even more preferably, the detection of nucleic acid sequences comprises amplifying and detecting at least one DNA or RNA sequence chosen among SEQ ID No 62 to 70.

Therefore, in a preferred embodiment, the present invention is drawn to a method for diagnosing a predisposition to oral cancer, or for diagnosing an oral cancer in a human subject, the method further comprising the steps of:

a) extracting total nucleic acid (genomic DNA and total RNA) of bacteria, virus, and human origins from the stabilized saliva sample,

b) incubating said genomic DNA and total RNA in the same tube with a thermostable enzyme with RNA-dependent Reverse Transcriptase activity and with DNA-dependent Polymerase activity, allowing the combination of RT and PCR in a single-tube reaction,

wherein said RT-PCR reaction is performed with primers and probes specific to:

i) human sequences selected from SSAT mRNA (SEQ ID No 62), H3F3A mRNA (SEQ ID No 63) and IL8 mRNA (SEQ ID No 64);

ii) sequences of bacteria selected from Capnocytophaga gingivalis (ATCC 33624, SEQ ID No 65), Prevotella melaninogenica (ATCC 25845, SEQ ID No 66), Streptococcus mitis (ATCC 15914, SEQ ID No 67) and Micrococcus luteus (ATCC 53598D, SEQ ID No 68),

iii) sequences of virus selected from human papillomavirus 16 (ATCC 45113, SEQ ID No 69) and human papillomavirus 18 (ATCC 45152, SEQ ID No 70);

and wherein the presence of at least one sequence of each i), ii) or iii), is indicative of a risk or a predisposition to oral cancer.

In a particular embodiment, the presence of at least one sequence of each i), ii) and iii), is indicative of a risk or a predisposition to oral cancer.

More specifically, the detection of the presence of a combination of at least one of said sequences iii), at least two of said sequences i) and at least two of said sequences ii) indicates that the human subject has an increased risk of developing oral cancer.

In one particular embodiment, the method comprises amplifying and detecting SEQ ID No 62 to 70.

In another particular embodiment, the invention is directed to a method for risk evaluation and diagnosis of oral cancer disease in a human subject comprising the steps of:

a) collecting a sample of crude saliva of said human subject in a sterile device,

b) stabilizing said sample by adding a solution comprising a guanidium salt, such as guanidinum thiocyanate, and/or ammonium sulfate, and/or sodium azide, and optionally exo and/or endonuclease inhibitors,

c) extracting total nucleic acid of bacteria, virus, and human origins from the previously obtained stabilized saliva sample,

d) precipitation and purification of total nucleic acids,

e) incubating the purified total nucleic acid with a thermostable enzyme with RNA-dependant reverse transcriptase activity and with DNA-dependant polymerase activity and polynucleotide primers under conditions which allow the reverse transcriptase activity of said thermostable enzyme to synthetise cDNA from the ribonucleic and amplification of genomic DNA and cDNA at a detectable level by Polymerase Chain Reaction,

f) detecting in an assay the amplified DNAs sequences by hybridization with one or more polynucleotide probes specific to :

i) human sequences selected from SSAT mRNA (SEQ ID No 62), H3F3A mRNA (SEQ ID No 63) and IL8 mRNA (SEQ ID No 64); or

ii) sequences of bacteria selected from Capnocytophaga gingivalis (ATCC 33624, SEQ ID No 65), Prevotella melaninogenica (ATCC 25845, SEQ ID No 66), Streptococcus mitis (ATCC 15914, SEQ ID No 67) and Micrococcus luteus (ATCC 53598D, SEQ ID No 68), or

iii) sequences of virus selected from human papillomavirus 16 (ATCC 45113, SEQ ID No 69) and human papillomavirus 18 (ATCC 45152, SEQ ID No 70).

Preferably, said method is performed by detecting the amplified DNAs sequences by hybridization with one or more polynucleotide probes specific to i), ii) and iii) .

In the above method, it is also contemplated to add a calibrated and know nucleic acid (DNA or RNA) to the vial used to collect saliva to measure the exact quantity of saliva collected by comparison with an external standard nucleic acid calibration curve.

Regarding step d), total nucleic acid present in the preservative reagent can be purified by a nucleic acid affinity resin.

In step e), the purified genomic DNA and total RNA are incubated in the same tube with a thermostable enzyme with RNA-dependent Reverse Transcriptase activity and with DNA-dependent Polymerase activity, allowing the combination of RT and PCR in a single-tube reaction, such as Tth DNA polymerase, and polynucleotide primers with nucleotides sequences that detect at least one of each i), ii) or iii) sequences.

According to the invention, polynucleotide primers and probes are natural nucleic acid or Peptide Nucleic Acid (PNA) or modified nucleic acid (superbase) which can hybridize to nucleic acid (DNA and RNA).

Step f) may further comprise the step of quantifying amplified DNA by comparison with quantified standard DNA or RNA and determining whether or not the nucleic acid is present, over present or overexpressed in the saliva sample.

In one particular embodiment, the invention relates to a method of measuring the presence of oral cancer risk factors in a human subject comprising the steps of:

- The recovery and preservation of total nucleic acid from crude saliva from degradation caused by nucleases,
- Optionally, the addition of a positive nucleic acid control for the full analytic process and exact quantification of the saliva amount,
- Nucleic acid extraction and concentration of total nucleic acid (genomic DNA and total RNA),
- A one-step Reverse Transcriptase Polymerase Chain Reaction : The purified genomic DNA and total RNA are incubated in the same tube with a thermostable enzyme with RNA-dependent Reverse Transcriptase activity and with DNA-dependent Polymerase activity, allowing the combination of RT and PCR in a single-tube reaction, such as Tth DNA polymerase, and polynucleotide primers with nucleotides sequences that detect *Capnocytophaga gingivalis* (ATCC 33624, Genbank Accession AF543295), *Prevotella melaninogenica* (ATCC 25845, Genbank Accession N° AJ555137), *Streptococcus mitis* (ATCC 15914, Genbank Accession N° AJ617805,) *Micrococcus luteus* (ATCC 53598D, Genbank Accession N° AM285006), human papillomavirus 16 (ATCC 45113, Genbank Accession N° EF422141), human papillomavirus 18 (ATCC 45152, Genbank Accession N° EF422111), SSAT mRNA (Genbank Accession N° NM002970), H3F3A mRNA (Genbank Accesion N° NM002107), [L8 mRNA (Genbank Accession N° NM000584).

Using one-step Real-time Reverse Transcriptase Polymerase Chain Reaction, the invention enables the user to perform a rapid RT-PCR and simultaneously detect and quantify the presence of total nucleic acid from bacteria, virus and human mRNA by monitoring fluorescence during real time polymerase chain reaction amplification without any risk of false positive due to opening tube between RT and PCR and from possible PCR product environmental contamination due to precedent amplification reactions in the same environment.

Beta actine mRNA (Genbank Accession Number X00351 - SEQ ID No 61) is useful as internal standard in step a) for calibration and quantification.

In a preferred embodiment, the polymerase with RNA-dependent Reverse Transcriptase activity and with DNA-dependent Polymerase activity is the Tth DNA polymerase.

Using total nucleic acid extraction and one-step Real-time Reverse Transcriptase Polymerase Chain Reaction in a same microfluidic cartridge, the invention enables the user to perform a point of care analysis usable in physician and dentist offices.

Alternatively, using one- step Reverse Transcriptase Polymerase Chain Reaction and a microarray, the invention enables the user to perform RT- PCR and detect and quantify the presence of total nucleic acid from bacteria, virus and human mRNA by measuring hybridization signal at the end of the RT- PCR and compare with external nucleic standard.

Preferably, the method of the invention use a microarray for diagnosing a predisposition to oral cancer in a human subject comprising probes sequences selected from the group consisting of SEQ ID No32 to SEQ ID N°60.

Advantageously, the polynucleotide primers and probes to amplify and detect at least one of each i), ii) or iii) sequences are preferably selected from the group consisting of SEQ ID No: 1 to SEQ ID No: 60.

Kit of parts associated with the methods herein disclosed are also disclosed, such as a kit to practice the above method, comprising primers and probes sequences to amplify and detect at least one of each of the sequences i), ii) or iii) depicted above, and including at least one of the following:

a) a sterile device to collect a saliva sample, optionally including a control nucleic acid, and a collect reagent,

b) a preservation reagent, for example a spray dry preservative reagent,

c) a resin having affinity for total nucleic acid,

d) a thermostable enzyme with RNA-dependant reverse transcriptase activity and with DNA-dependant polymerase activity and polynucleotide.

As mentioned before, the collect reagent is a dilution buffer which is preferably citrate buffer.

The kit comprises a preservation reagent, which is preferably a buffer comprising a salt such as guanidinum thiocyanate, and/or ammonium sulfate and/or sodium azide, and optionally exo and/or endonuclease inhibitors. The sodium azide salt is employed preferably at a concentration range between 20mM and 100mM, more preferably around 40mM. Other salts, such as guanidium thiocyanate and /or ammonium sulphate, are added at a concentration of 4M.

The preservation reagent is provided in dry format in a sterile plastic tube under vacuum, which can draw up the crude saliva associated with the dilution buffer.

Primers and probes sequences to amplify and detect at least one of each of the sequences i), ii) or iii) depicted above are further defined as comprising polynucleotide primers for synthesizing cDNA by reverse transcription, polynucleotide primers for amplifying genomic DNA and cDNA by polymerase chain reaction and polynucleotide probes for detecting amplified DNA.

In the diagnostic kits herein disclosed, the reagent can be provided in the kits, with suitable instructions and other necessary reagents in order to perform the methods here disclosed. Instructions, for example written or audio instructions, on paper or electronic support such as tapes or CD-roms, for carrying out the assay, will be usually included in the kit.

In first aspect, the present invention is drawn to *in vitro* method of diagnosing oral cancer in a human subject, the method comprising stabilizing a crude saliva sample from said human subject, and analyzing a volatile fraction extracted from said stabilized saliva by detecting in said volatile fraction at least one biochemical organic compound, wherein the detection of at least one biochemical organic compound is indicative of a risk of developing an oral cancer.

From a chemistry point of view, biochemical organic compounds are the members of a large class of chemical compounds whose molecules contain carbon. They can be antigens, carbohydrates, enzymes, hormones, lipids, fatty acids, neurotransmitters, nucleic acids, proteins, peptides and amino acids, vitamins, fats and oils.

Among all the known organic compounds, "Volatile organic compounds" (VOC) are meant to designate any organic compound that is volatile, i.e. that have a high vapor pressure or low boiling point, and can therefore evaporate at normal temperature and pressure. These compounds are often regulated by governments. For example, in European Union, a "Volatile Organic Compound" is any organic compound having an initial boiling point less than or equal to 250°C measured at a standard atmospheric pressure of 101,3 kPa.

In the context of the invention, the "volatile fraction" is recovered from the heating of a crude saliva sample. Preferably, said volatile fraction is extracted from crude saliva sample by heating said saliva sample for at least 10 minutes, preferably 20 minutes and more preferably 30 minutes at a temperature comprised between 30°C and 50°C, preferably 40°C. During this time, the volatile fraction is taken away from the sample by using a solid-phase microextraction (SPME) with a carboxen/polydimethylsiloxane coated fiber (CAR/PDMS fiber).

Therefore, in one embodiment of the present invention, the volatile biochemical organic compounds are extracted with a CAR/ PDMS fiber coating during at least, preferably 20 minutes, and even more preferably 30 minutes from a saliva sample that is simultaneously heated at a temperature comprised between 30°C and 50°C, and preferably at about 40°C. The desorption temperature of the fiber is comprised between 250°C and 300°C, and is preferably of about 280°C.

Solid- phase microextraction (SPME) is a patented sample preparation technique based on the adsorption of analytes directly from an aqueous sample onto a coated, fused- silica fiber. This sampling technique is fast, easy to use and eliminates the use of organic solvents (Mills G et al, Journal of Chromatography 2000; Song C et at, Lung Cancer 2009) .

In this technology, the CAR/PDMS fibers are often used for detecting trace level of volatile compounds, and are therefore well-known from the man skilled in the art (Garcia-Esteban M et al, Talanta 2004).

Preferably, the detection of said biochemical organic compound is performed by using a chromatograph in gas phase coupled to a mass spectrometer.

In the context of the invention, a biochemical compound is "detected" when the expression level of said compound is at least superior to 1.5 fold the mean expression level of said compound in the normal population.

By applying the method of the invention, some biochemical compounds were shown to be highly overexpressed in the volatile fraction of human subjects suffering from oral cancer and were therefore found to be acute and sensitive diagnostic and/or prognostic tool of oral cancer. Importantly, none of these compounds can be detected in the fluid fraction of saliva, highlighting the necessity to study the volatile fraction of saliva in this case.

In a preferred embodiment, the present invention is drawn to a method of diagnosing a risk or a predisposition to oral cancer in a human subject by analyzing the content in biochemical compounds in the volatile fraction of stabilized samples of saliva, wherein the detection of at least one of the biochemical organic compound selected in the group consisting of: 2, 3- pentanedione (CAS number 600- 14- 6), 3- methylthiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3), 2- methyl- 2- butenal (E) (CAS number 497- 03- 0), phenol (CAS number 108- 95- 2), butanal (CAS number 123- 72- 8), methylbutanone (CAS number: 563- 80- 4), 2- methyl- 2- butene (CAS number 513- 35- 9), 2- methyl- 1- propene (CAS number 115- 11- 7) and (*cis*) 1, 2 dimethyl- cyclopropane (CAS number: 930- 18- 7) is indicative of a risk or predisposition to oral cancer.

Indeed, the below- presented results have shown that the detection of at least one of the following compounds: 2, 3- pentanedione (CAS number 600- 14- 6), 3- methylthiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3), 2- methyl- 2- butenal (E) (CAS number 497- 03- 0) in the volatile fraction of saliva of a human subject indicates that said human subject has a high risk of being predisposed to develop oral cancer or has a high risk of being developing an oral cancer. More precisely, the detection of at least one of the above- mentioned biochemical organic compounds indicates that the tested human subject has a risk superior to 60% of developing an oral cancer; the detection of at least two of the above- mentioned biochemical organic compounds indicates that the tested human subject has a risk superior to 70% of developing an oral cancer, and the detection of at least three of the above- mentioned biochemical organic compounds indicates that the tested human subject has a risk superior to 80% of developing an oral cancer; the detection of at least four of the above- mentioned biochemical organic compounds indicates that the tested human subject has a risk superior to 90% of developing an oral cancer. On the contrary, when at least one of the particulate biochemical organic compounds disclosed above is not detected in the saliva sample of a human subject, it means that said human subject has a low risk of developing an oral cancer.

For example, as shown in the example 10, the detection of the biochemical organic compounds of the group comprising: hexanitrile, 2, 3- pentanedione, 3- methylthiophene and acetone in the volatile fraction of saliva of a human subject indicates that said human subject has a risk superior to 97% of developing an oral cancer, and is therefore predisposed to develop an oral cancer, or is developing an oral cancer.

Also, as shown in the example 11, the detection of the mRNA sequence of H3F3A (SEQID No 63), SSAT (SEQ ID No 62) and of the biochemical organic compounds hexanenitrile, 2, 3- pentanedione, 3- methylthiophene and acetone in the saliva of a human subject enables to detect 100% of cancer cases in a tested population. Therefore, in a preferred embodiment, the detection of the mRNA sequence of H3F3A (SEQID No 63), SSAT (SEQ ID No 62) and of the biochemical organic compounds hexanenitrile, 2, 3- pentanedione, 3- methylthiophene and acetone in the saliva of a human subject indicates that said human subject is developing an oral cancer.

Also, as examplified hereunder, the method of the present invention can rely on the detection of 3- methyl- 2- pentanone, methyl butanone, butanal, hexanal, hexanenitrile, 1- propanol 2- propanol, (cis) 1, 2- dimethyl cyclopropane, phenol, and 2, 3- butanedione in order to prognose or to diagnose an oral cancer in a human subject.

In another embodiment, the present invention is drawn to a method of diagnosing a risk or a predisposition to oral cancer in a human subject, wherein the detection of at least one biochemical organic compound in the volatile fraction of saliva of a human subject indicates that said human subject is not predisposed to develop an oral cancer or is not develop ing an oral cancer.

The compounds 2- methyl- 2- butene (CAS number 513- 35- 9), 2- methyl- l- propene (CAS number 115- 11- 7) and *(cis)* 1, 2- dimethyl cyclopropane (CAS number 930- 18- 7) are overexpressed in the volatile fraction of healthy human subject and are absent in the volatile fraction of patients suffering from oral cancer. Therefore, these compounds can serve as "healthy biochemical markers". The detection of at least one, preferably two of these biochemical organic compounds indeed indicates that the human subject has a low risk of being predisposed of being developing an oral cancer.

Moreover, the detection of particulate biochemical organic compounds such as benzaldehyde, acetone, 2, 3- pentanedione, on a one hand, and the absence of other particulate biochemical compounds such as 2- methyl- 2- butene, 2- methyl- 1- propene, and/or *(cis)* 1, 2- dimethyl cyclopropane on the other hand, enables to diagnose oral cancer with a high sensitivity (at least 93%, see example 10) .

It is noteworthy that most of these biochemical compounds have never been identified so far in the saliva. Moreover, none of them have been related so far with cancer predisposition, and *a fortiori* with oral cancer predispositon.

Therefore, the present invention is also drawn to the use of the detection of at least one compound chosen among: 2, 3- pentanedione (CAS number 600- 14- 6), 3- methylthiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3), 2- methyl- 2- butenal (E) (CAS number 497- 03- 0), 2- methyl- 2- butene (CAS number 513- 35- 9), 2- methyl- l- propene (CAS number 115- 11- 7) and *(cis)* 1, 2- dimethyl cyclopropane (CAS number 930- 18- 7) in a diagnostic test to assess the risk of developing an oral cancer in a human subject.

In a particulate embodiment, the present invention is therefore drawn to a method of diagnosing a risk or a predisposition to oral cancer in a human subject, comprising the steps of:

a) collecting a sample of crude saliva of said human subject in a sterile device,

b) stabilizing said sample by adding a solution comprising a salt, such as guanidinum thiocyanate, ammonium sulfate and/or sodium azide,

c) extracting the volatile fraction from said stabilized sample by heating it for at least 10 minutes at 40°C and using for example Solid-phase Microextraction (SPME) to take away the volatile fraction,

d) detecting at least one biochemical organic compound by using for example a chromatograph in gas phase coupled to a mass spectrometer,

wherein the detection of at least one, preferably at least two, and more preferably at least three biochemical organic compound(s) is indicative of a risk or a predisposition to oral cancer.

In a preferred embodiment, the at least one, preferably at least two, and more preferably at least three detected biochemical organic compound is (are) chosen among: 2, 3- pentanedione (CAS number 600- 14- 6), 3- methylthiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3) and , 2- methyl- 2- butenal (E) (CAS number 497- 03- 0), phenol (CAS number 108- 95- 2), butanal (CAS number 123- 72- 8), methylbutanone (CAS number: 563- 80- 4), 2- methyl- 2- butene (CAS number 513- 35- 9), 2- methyl- 1- propene (CAS number 115- 11- 7) and *(cis)* 1, 2 dimethyl- cyclopropane (CAS number: 930- 18- 7) .

In a preferred embodiment, the detection of the at least one detected biochemical compound chosen among 2, 3- pentanedione (CAS number 600- 14- 6), 3- methylthiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3), and 2- methyl- 2- butenal (E) (CAS number 497- 03- 0) is indicative of a high risk of developing cancer as compared to a normal healthy population.

On the contrary, when at least one of the biochemical compounds: 2- methyl- 2- butene (CAS number 513- 35- 9), 2- methyl- l- propene (CAS number 115- 11- 7) or (*cis*) 1, 2- dimethyl cyclopropane (CAS number 930- 18- 7) is detected, it is indicative of a poor risk of developing oral cancer, i.e. a risk inferior to 10%, preferably 5 % to develop cancer as compared to a normal healthy population.

In a second aspect, the present invention is drawn to the use for practicing a method of diagnosing a risk or a predisposition to oral cancer based on the volatile fraction of saliva of a kit comprising:

a) A sterile device to collect a saliva sample, optionally containing a collect reagent

b) A preservation reagent,

c) At least one electronic sensor,

d) Optionally, a control molecular marker.

As mentioned before, the collect reagent is a dilution buffer which is preferably a citrate buffer.

The kit comprises a preservation reagent, which is preferably a buffer comprising a salt capable of reducing the vapour tension of volatile compounds without allowing the degradation of said compounds. This salt is preferably a salt such as guanidinum thiocyanate, and/or ammonium sulfate and/or sodium azide. This sodium azide is employed preferably at a concentration range between 20mM and 6M, preferably between around 10mM and 100mM, more preferably around 40mM. Other salts, such as guanidium thiocyanate and /or ammonium sulphate, are added at a concentration of 4M.

In one embodiment of the present invention, the preservation reagent is provided in a dry format in a sterile plastic tube under vacuum, which can draw up the saliva associated with the dilution buffer.

In the context of the invention, the device used to detect the organic compounds in the collected volatile fraction of saliva is an electronic sensor, for example electronic noses, JPL electronic noses, FET-type Bioelectronic noses, alpha mos). These technologies are now widely used and therefore known from the man skilled in the art (Cho S.M., Sensors and Actuators 2006).

Using specific electronic sensors for the identification of the targeted volatile compounds in a specific platform (electronic nose), the invention enables the user to perform a specific analysis platform or a point of care analysis usable in physician and dentists offices.

In one embodiment of the invention the control molecular markers are chosen among: 1-bromobutane, 1-bromobenzene and 1,4-dibromobenzene.

**Example 1: Stabilization of crude samples of saliva**

Raw saliva is collected with a medical device which makes easier the collection of a large volume of saliva (up to 2 ml) following by a stabilization of the saliva biomarkers (AND, ARN, peptides, volatile compound).

4 ml of saliva extraction solution is then swallowed up to 2 minutes for collection of 2 ml of saliva.

The saliva extraction solution contains:

- FD&C yellow n°5 (tartrazine)

- Citrate buffer (39 mM)

The 2 ml of diluted saliva (in 4 ml) are then transferred in tubes containing lyophilized sodium azide for biomarker stabilization. The final sodium azide concentration is about 40mM.

2 tubes are prepared for each sample. A tube is intended for the "genetic" analyzes (tube 1) and the other tube (tube 2) is used for the analyzis of the volatile compounds.

The tubes can be transported without control of temperature during 10 days before being analyzed.

**Example 2: Simultaneous determination and quantification in stabilized saliva of the presence of bacteria, virus and human mRNA to assess risk factor of oral cancer.**

0, 250 to 3 ml of the solution from tube 1 is prepared and total nucleic acids are extracted by centrifiltration on a silica membrane without DNAse step. Up to 1.5 mg of total nucleic acid are extracted and purified from 1 ml of stabilized saliva.

Isolation of high-quality DNA and RNA from whole saliva samples is difficult because under ambient conditions, expression and quantification profile are unstable on a timescale below few minutes. This instability is the result of metabolic activity of bacteria, nutrients dependant, concentration in nucleases and limited turnover of RNA in that environmental conditions. In order to render the nucleic acid inaccessible to nuclease, we used a preservation buffer which comprises a salt for membrane lysis of bacteria as well as human cells and precipitates the extracted nucleic acid in the sample along with the cellular protein. We used in this regard a guanidinium salt such as guanidinum thiocyanate and or ammonium sulfate associated or not with a ribonuclease inhibitor (EDTA < 10 mM).

Preferred biological targets to be detected in crude saliva are *Capnocytophaga gingivalis* (ATCC 33624, Genbank Accession N° AF543295), *Prevotella melaninogenica* (ATCC 25845, Genbank Accession N° AJ555137), *Streptococcus mitis* (ATCC 15914, Genbank Accession N° AJ617805,) *Micrococcus luteus* (ATCC 53598D, Genbank Accession N° AM285006), human papillomavirus 16 (ATCC 45113, Genbank Accession N° EF422141), human papillomavirus 18 (ATCC 45152, Genbank Accession N° EF422111), SSAT mRNA (Genbank Accession N° NM002970), H3F3A mRNA (Genbank Accesion N° NM002107), IL8 mRNA (Genbank Accession N° NM000584).

Beta actine mRNA (Genbank Accession N° X00351) is used as internal control for calibration and quantification.

After obtaining bacteria, virus, human cells and extracellular human total nucleic acid from a saliva sample in a sterile device, the preservation buffer is added into the saliva sample at room temperature. The buffer is preferably able to open prokaryotic and eukaryotic cells, associated with preservation of total nucleic acids by action of blocking nucleases activities and precipitation of total nucleic acids. The preservation reagent is provided in dry format in a sterile polyethylene terephtalate (PET) plastic tube. Stabilizing reagent is calibrated to draw up to 3 ml of saliva associated with a dilution buffer. Calibrated and known nucleic acids (DNA or RNA), for example can be added to the collect vial in order to measure the exact quantity of saliva collected and analyzed by comparison with external standard calibration curve obtained after extraction and Reverse Transcriptase PCR quantification (some of the biotargets measured are expressed in genomic quantity per ml of saliva). Calibrated and known nucleic acids (DNA or RNA) added to the collect vial will also permit to verify the global performance of the full analytical process. Total nucleic acids are purified with a Nucleic Acid affnity resin. Our preferred system used coated paramagnetic beads compatible with the guanidinium salt having a rate of recovery of total nucleic acids from crude sample up to 90% and no selection between RNA and DNA.

The basic RT PCR process is carried out as follows. The RNA present in the total nucleic acid contained in the sample may be first reverse transcribed into cDNA (using enzyme like Tth DNA polymerase as purified enzyme and a oligonucleotide or PNA or modified oligonucleotide), and then denatured, using physical means, which are known to those of skill in the art. A preferred physical means for strand separation involves heating the nucleic acid until it is completely (>99%) denatured. Methods for the amplification of RNA targets using a thermostable DNA polymerase are described in W09109944 incorporated herein by reference. The denatured DNA strands are then incubated in the same tube with the selected oligonucleotide primers under hybridization conditions, conditions which enable the binding of the primers to the single DNA strands. As known in the art, the primers are selected so that their relative positions along a duplex sequence are such that an extension product synthesized from one primer, when it is separated from its complement, serves as a template for the extension of the other primer to yield a replicate chain of defined length. The primer must be sufficiently long to prime the synthesis of extension products in the presence of the agent for polymerization. The exact length of the primers will depend on many factors, including temperature, source of the primer and use of the method.

Preferred oligonucleotide primers for use in the present invention are selected from the group consisting of SEQ ID No 1 to SEQ ID No 31.

Template- dependent extension of the oligonucleotide primer (s) is then catalyzed by the polymerizing agent (in the presence of adequate amounts of the four deoxyribonucleoside triphosphates (dATP, dGTP, dCTP, and dTTP or analogs), in a reaction medium which is comprised of the appropriate salts, metal cations, and pH buffering system. The products of the synthesis are duplex molecules consisting of the template strands and the primer extension strands, which include the target sequence. These products, in turn, serve as templates for another round of replication. In the second round of replication, the primer extension strand of the first cycle is annealed with its complementary primer; synthesis yields a"short"product which is bounded on both the 5'- and the 3'- ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method is performed in a fashion where all of the reagents are added simultaneously, in one step. In a preferred method, the RT PCR reaction is carried out as an automated process which utilizes a thermostable enzyme like Tth. In a preferred method, the RT PCR reaction is performed in a types of thermocycler having capability for reading at least 4 different florescence dyes and developed/manufactured for real time PCR assays and commercial use.

Those skilled in the art will also be aware of the problems of contamination of a PCR by the nucleic acid from bacteria previously present in water used for buffer and resulting in non specific amplification or background. Methods to reduce these problems are provided by using adequate buffer, reagents and enzymes to avoid nucleic acid strand fragments with a size higher than 100 bp. All reagents used in the RT PCR reaction have to be processed before using. During amplification by PCR, the target polynucleotides may be detected directly by hybridization with a probe polynucleotide which forms a stable hybrid with the target sequence under high stringency to low stringency hybridization and washing conditions. Probes are typically labeled with non-radioactive labeling systems, such as fluoresceins and derivated systems.

Reverse Transcriptase activity and with DNA-dependent Polymerase activity, allowing the combination of RT and PCR in a single-tube reaction, such as Tth DNA polymerase or an enzyme like Tth DNA polymerase, and polynucleotide primers with a nucleotide sequence selected from the group consisting of SEQ ID No 2 SEQ ID No 4 SEQ ID No 5 SEQ ID No 8 SEQ ID No 10 SEQ ID No 12 SEQ ID No 14 SEQ ID No 16 SEQ ID No 18 SEQ ID No 20 SEQ ID No 21 SEQ ID No 23 SEQ ID No 25 SEQ ID No 27 SEQ ID No 29 and SEQ ID No 31 under conditions which allow hybridization of the polynucleotide to the ribonucleotide target region and Reverse Transcriptase activity of the said polymerase, or enzyme like Tth, for cDNA synthesis; and (c) amplified the cDNAs formed to a detectable level by Polymerase Chain Reaction with said polymerase enzyme like Tth DNA polymerase and polynucleotide primers and probes with a nucleotide sequence selected from the group consisting of SEQ ID No 1 to SEQ ID No 60.

More particularly, the preferred combination of primers and probes used to detect bacteria, virus and human mRNA consists of the sequences:

***Prevotella melaninogenica*** (ATCC 25845, Genbank Accession N° AJ 555137)

Seq ID No 1 + Seq ID No 2 +Seq ID No 32, or Seq ID No 1 + Seq ID No 2 + Seq ID No 49, or Seq ID No 3 + Seq ID No 2 + Seq ID No 49, or Seq ID No 1 + Seq ID No 4 + Seq ID No 32, or Seq ID No 3 + Seq ID No 5 + Seq ID No 49

***Streptococcus mitis*** (ATCC 15914, Genbank Accession N° AJ617805,)

Seq ID No 6 + Seq ID No 8 +Seq ID No 50 or Seq ID No 7 + Seq ID No 8 + Seq ID No 33 or Seq ID No 7 + Seq ID No 8 + Seq ID No 51

***Capnocytophaga gingivalis*** (ATCC 33624, Genbank Accession AF543295)

Seq ID No 9 + Seq ID No 10 +Seq 10 No 34 or Seq ID No 9 + Seq ID No 10+Seq ID No 52

***Micrococcus luteus*** (ATCC 53598D, Genbank Accession N° AM285006)

Seq ID No 11 + Seq ID No 12 +Seq ID No 35, or Seq ID No 11 + Seq ID No 12 + Seq ID No 53

**Human papillomavirus 16** (ATCC 45113, Genbank Accession N° EF422141),

Seq ID No 13 + Seq ID No 14 +Seq ID No 36

**Human papillomavirus 18** (ATCC 45152, Genbank Accession N° EF422111)

Seq ID No 15 + Seq ID No 16 +Seq ID No 37

**Beta actine mRNA** (Genbank Accession N° X00351)

Seq ID No 17 + Seq ID No 18 +Seq ID No 38 or Seq ID No 17 + Seq ID No 18 +Seq ID No 39 or Seq ID No 17 + Seq ID No 18 +Seq ID No 54 or Seq ID No 17 + Seq ID No 18 +Seq ID No 55 or Seq ID No 19 + Seq ID No 20 + Seq ID No 40 or Seq ID No 19 + Seq ID No 20 + Seq ID No 41 or Seq ID No 19 + Seq ID No 20 + Seq ID No 56

**SSAT mRNA** (Genbank Accession N° NM002970)

Seq ID No 22 + Seq ID No 23 +Seq ID No 42 or Seq ID No 22 + Seq ID No 23 + Seq ID No 57 or Seq ID No 24 + Seq ID No 25 + Seq ID No 43

**H3F3A mRNA** (Genbank Accesion N° NM002107

Seq ID No 26 + Seq ID No 27 +Seq ID No 44 or Seq ID No 26 + Seq ID No 27 + Seq ID No 58 or Seq ID No 26 + Seq ID No 27 + Seq ID No 45 or Seq ID No 28 + Seq ID No 29 + Seq ID No 46 or Seq ID No 28 + Seq ID No 29 + Seq ID No 59

**IL8 mRNA** (Genbank Accession N° NM000584)

Seq ID No 30 + Seq ID No 31 +Seq ID No 47 or Seq ID No 30 + Seq ID No 31 + Seq ID No 48 or Seq ID No 30 + Seq ID No 31 + Seq ID No 60

Also, according to the invention, the preferred probes used to detect bacteria, virus and human mRNA fixed on a microarray surface are selected from the group consisting of SEQ ID No 32 to SEQ ID No 60.

**Example 3: Analysis of genetic markers in the fluid fraction by more than 2 separate steps**

1- The saliva sample (up to 1000 μL) is mixed with a diluting buffer (sterile nuclease free reagent) and passed through a sterile polyethylene terephtalate (PET) plastic tube. Preservative reagent and a known nucleic acid (pure synthetic ribonucleotide) are calibrated to draw up to 3 ml of saliva associated with the dilution buffer. Full process should be realized in less than 2 minutes. This process permits immediate preservation of total nucleic acids at room temperature for up to 10 days to allow transportation delays via regular mail to laboratory.

2- Lysis at laboratory, transfer up to 1000 μl of liquid from the PET plastic into a 2 mL sterile tube with up to 1 mL of lysis buffer and then incubate at 35°C +/- 2°C for up to one hour.

3- The lysat is processed for total nucleic acids purification with magnetic silica or polystyrene beads or funnel-design having silica membrane in mini prep spin columns able to concentrate circulating nucleic acid from plasma. The elution volume is up to 100 μl. 5- 2 μL (up to 5 μL) of pure nucleic acids extract is used for the one step real time RT-PCR (RotorGene) with enzyme like Tth and the following program with Taqman Probe: I: Reverse transcription 61 °C/ 20 min (20°C/sec) II : Denaturation 95°C/ 30 secondes (20°C/sec) III : PCR (35 cycles) 95°C/ 5 seconds (20°C/sec) 60°C/ 30 seconds (20°C/sec) . The emitted fluorescence is measured at the end of the 60°C

**Example 4: Analysis of the fluid fraction of saliva sample using microarrays**

1- The saliva sample (up to 1000 μL) is mixed with a diluting buffer (sterile nuclease free water) and passed through a sterile polyethylene terephtalate (PET) plastic tube. Preservative reagent and a known nucleic acid (pure synthetic ribonucleotide) are calibrated to draw up to 3 ml of saliva associated with the dilution buffer. Full process should be realized in less than 2 minutes. This process permits immediate preservation of total nucleic acid at room temperature for up to 10 days to allow transportation delays via regular mail to laboratory.

2- Lysis at laboratory , transfer up to 1000 μl of liquid from the PET plastic into a 2 mL sterile tube with up to 1 mL of lysis buffer and then incubate at 35°C +/- 2°C for up to one hour.

3- The lysat is processed for total nucleic acids purification with magnetic silica or polystyrene beads or funnel-design having silica membrane in mini prep spin columns able to concentrate circulating nucleic acid from plasma. The elution volume is up to 100 μl. 5- 2 μL (up to 5 μL) of pure nucleic acids extract is used for the one step RT- PCR with enzyme like Tth and the following program without Probes: I: Reverse transcription 61 °C/ 20 min (20°C/sec) II : Denaturation 95°C/ 30 secondes III : PCR (35 cycles) 95°C/ 20 seconds, 60°C/ 20 seconds, 72°C/ 30 seconds.

Primers are Cy5 fluorescence labeled. Probes have been coated on surface of a PET slide using oligonucleotide arms.

5 μl of amplified DNA is mixed in 30 μL of hybridization buffer and incubated 2 minutes at 95°C. 25μl of the denatured amplified DNA is hybridized on the microarray at room temperature during 10 minutes, briefly washed in two washing buffer. The microarray is dried and fluorescence is measured on a scanner and compared to standards.

**Example 5: Analysis of the fluid fraction of saliva by using real time PCR point of care instrument associated to a microfluidics cartridge able to extract and amplify total nucleic acids (GeneXpertTM solution from Cepheid inc or LiatTM system from Iquum inc).**

1- The saliva sample (up to 1000 μL) is mixed with a diluting buffer (sterile nuclease free water) and passed through a sterile polyethylene terephtalate (PET) plastic tube. Stabilizing reagent and a know nucleic acid (pure synthetic ribonucleotide) are calibrated to draw up to 3 ml of saliva associated with the dilution buffer.
Full process should be realized in less than 2 minutes.
This process permits immediate preservation of total nucleic acid at room temperature for up to 10 days to allow transportation delays via regular mail to laboratory or direct analysis in the dental of physician office.

2- Up to 3 ml or 5 ml of saliva in stabilizing reagent is transfered in the microfluidics cartridge chamber having lysis buffer able to process for total nucleic acid purification with polystyrene beads and/or associated to physical and/or mechanical lysis. Without technician intervention, 2 μL (up to 5 μL) of pure nucleic acids extracted are transferred in the one step real time RT-PCR chamber having enzyme like Tth and associated mix PCR reagents and the following program

- with Taqman Probe: I: Reverse transcription 61 °C/20 min (20°C/sec) II : Denaturation 95°C/30 secondes (20°C/sec) III : PCR (35 cycles) 95°C/5 seconds (20°C/sec) 60°C/30 seconds (20°C/sec). The emitted fluorescence is measured at the end of the 60°C.

**Example 6: Evaluation of the overexpression of several genetic marker in the saliva of oral cancer patients by quantitative PCR**

1- The saliva sample (up to 1000 μL) is mixed with a diluting buffer (sterile nuclease free water) and passed through a sterile polyethylene terephtalate (PET) plastic tube. Preservative reagent and a known nucleic acid (pure synthetic ribonucleotide) are calibrated to draw up to 3 ml of saliva associated with the dilution buffer. Full process should be realized in less than 2 minutes. This process permits immediate preservation of total nucleic acid at room temperature for up to 10 days to allow transportation delays via regular mail to laboratory.

2- Lysis at laboratory , transfer 250 μl of liquid from the PET plastic into a 2 mL sterile tube with Proteinase K and then incubate at 56°C +/- 2°C for up to one hour. Incubate the pre-lysed sample with a lysis buffer, 10 minutes at 70°C +/- 2°C.

3- The lysat is processed for total nucleic acids purification with funnel-design having silica membrane in mini prep spin columns able to concentrate nucleic acid. The elution volume is up to 100μL. 2 μL (up to 5 μL) of pure nucleic acids extract is used for one step RT-PCRs with enzyme like Tth and for one step PCRs with enzyme like DNA Polymerase. The following program is peformed for the one step RT-PCR : I: Reverse transcription 48°C/15 min II : Denaturation 95°C/10 minutes III : PCR (40 cycles) 95°C/15 seconds, 60°C/ 60 seconds. The following program is peformed for the one step PCR : I : Denaturation 95°C/30 seconds III : PCR (45 cycles) 95°C/10 seconds, 60°C/ 10 seconds, 72°C/35 seconds.

One step PCRs, using the primers SEQID N°1, SEQID N°2, SEQID N°7, SEQID N°8, SEQID N°9 and SEQIDN° 10 allow to amplified the DNA from *Prevotella melaninogenica, Streptococcus mitis* and *Capnocytophaga gingivalis* respectively. The detection and quantification system is a Syber Green sytem or a a probe sytem using the SEQID N°32 or SEQID N°49 for *Prevotella melaninogenica* quantification, SEQID N° 33 or SEQID N°51 for *Streptococcus mitis* quantification and SEQID N° N° 34 or SEQID N°52 for *Capnocytophaga gingivalis* quantification.

One step RT-PCRs, using the primers SEQID N°26, SEQID N°27 or SEQID N° 28, SEQID N°29 and SEQID N°22 and SEQID N°23 allow to amplify H3F3A and SSAT mRNA respectively. The detection and quantification system could a Syber Green sytem or a probe sytem using the SEQID N°44 or SEQID N°58 or SEQID N°45 or SEQID N°46 or SEQID N°59 for H3F3A quantification and SEQID N°42 or SEQID N°57 for SSAT quantification. Each assay is confirmed using commercial probe kits (ABI) that amplified the H3F3A gene NM005324 on the exon 4 and the SSAT gene NM 002970 on the exons 3 and 4.

The results obtained for the patients from the oral cancer population versus the healthy individuals are presented in table 1.

**Table 1: overexpression of genetic biomarker in oral cancer population**

| Genetic biomarker name | Overexpression in oral cancer population |
|---|---|
| *Streptococcus mitis* | 2,80 X |
| *Capnocytophaga gingivalis* | 2.00X |
| *Prevotella melaninogenica* | 2.30X |
| SSAT | 2,63X |
| H3F3A | 2,56X |

It has been concluded from these data that the "detection" of a certain genetic marker (DNA or RNA) in a saliva sample of a patient means that said sample contains at least 2 fold the amount of said marker in the normal population.

Importantly, the tumorigenic status of 11 samples among 17 has been detected using the genetic markers SSAT, of H3F3A and the sequence of the bacteria *Streptococcus mitis.* Therefore, it can be conclude that the detection of these at least three genetic marker in the saliva of a human subject enables to diagnose an oral cancer with a sensibility of 64%. In other word, the detection of the mRNA of SSAT, the mRNA of H3F3A and the bacteria sequence of *Streptococcus mitis* indicates that the human subject has a risk superior to 60% of developping an oral cancer.

Accordingly, IL8 is not considered to be a significant marker for oral cancer in saliva.

**Example 7: Analysis of organic volatile compounds in the volatile fraction of saliva**

It is known for a while that volatile compounds can be extracted from fluidic samples from oral cavity giving the possibility to explore the saliva as material to be analyzed for pathogenic diseases (Volozhin et al. Stomatologiia (mosk), 2001; 80 (1) : 9- 12) .

In the present case, 1 ml of saliva solution is placed in a glass vial with 10 μL of the standard solution with 1 ppm of three (3) standards (1- bromobutane, 1- bromobenzene and 1.4- dibromobenzene; final solution with 1 ppm prepared in pure water) .

The samples are placed at room temperature during at least 1 hour before analyzes. The sample is heated at 40°C during 10 minutes then the extraction of the volatile compounds is carried out at 40°C, using a CAR/PDMS fiber (SPME fiber assembly CAR/PDMS of 75μm (Supelco, Bellefont, PA, USA)), during 30 minutes. Then the analysis was performed using GC/MS. The GC injection port temperature is 280°C. The injection of the volatile molecules in GC/MS is carried out by thermal desorption of the fiber at 280°C. The separation of the volatile compounds was led with a nonpolar capillary column. The column temperature program was: initial temperature of 40°C for 5 min, then increase at 3°C/min to 230°C for 2 min. The mass spectra are measured by electronic impact at 70 e.V.

The identification of the volatile molecules is obtained by:

- comparison of the experimental indices of retention to those of the internal data bank
- comparison of the experimental spectra to those of the bank Wiley 275K.

The results of the exhaustive analysis of all the volatile organic compounds found in the volatile fraction of human saliva are reported on table 2.

**Table 2: Organic volatile compounds in volatile fraction of human saliva**

| Molecule name | CAS number |
|---|---|
| 1,2-dichlorobenzene | 95-50-1 |
| disulfure de carbone | 137-26-8 |
| benzenecarboxylic acid | 65-85-0 |
| heptanoic acid | 111-14-8 |
| nonanoic acid | 112-05-0 |
| octanoic acid | 124-07-2 |
| pentanoic acid | 109-52-4 |
| 2-propenal, 2-methyl- | 78-85-3 |
| butanal | 123-72-8 |
| butanal, 3-methyl- | 590-86-3 |
| decanal | 112-31-2 |
| ethanal | 75-07-0 |
| heptanal | 111-71-7 |
| hexanal | 66-25-1 |
| hexanal, 2-ethyl- | 123-05-7 |
| nonanal | 124-19-6 |
| octanal | 124-13-0 |
| propanal, 2-methyl- | 78-84-2 |
| valeraldehyde, 4,4-dimethyl-2-methylene | 5375-28-0 |
| 7-oxabicyclo[4.1.0]heptane, 1-methyl- | 1713-33-3 |
| cyclopentane, methyl- | 96-37-7 |
| decane | 124-18-5 |
| hexane | 110-54-3 |
| methylcyclohexane | 108-87-2 |
| nonane | 111-84-2 |
| pentane, 2,2,4-trimethyl- | 540-84-1 |
| pentane, 2-methyl- | 107-83-5 |
| pentane, 3-methyl- | 96-14-0 |
| benzene | 71-43-2 |
| benzene, ethyl- | 100-41-4 |
| Butylated Hydroxytoluene | 128-37-0 |
| dehydro p-cymene | 1195-32-0 |

(continued)

| Molecule name | CAS number |
|---|---|
| dibenzofuran | 132-64-9 |
| m-xylene | 108-38-3 |
| o-xylene | 95-47-6 |
| p-cymene | 99-87-6 |
| styrene | 100-42-5 |
| toluene | 108-88-3 |
| 1-hexene, 3,5,5-trimethyl- | 4316-65-8 |
| 2-hexene, 2,5,5-trimethyl- | 40467-04-7 |
| butanoic acid, 2-methyl-, ethyl ester | 7452-79-1 |
| butanoic acid, 3-methyl-, ethyl ester | 108-64-5 |
| methyl thiolacetate | 1534-08-3 |
| ethyl butanoate | 105-54-4 |
| ethyl propanoate | 105-37-3 |
| ethyl-N-methylcarbamate | 105-40-8 |
| methylacetate | 79-20-9 |
| 1,3-dioxolane, 2-methyl- | 497-26-7 |
| tert-butyl ethyl ether | 637-92-3 |
| bromoethane | 74-96-4 |
| bromomethane | 74-83-9 |
| chlorobutanol | 57-15-8 |
| dibromomethane | 74-95-3 |
| dichloromethane | 75-09-2 |
| heptane, 3-bromo- | 1974-05-6 |
| hexane, 1-chloro- | 544-10-5 |
| hexane, 3-chloro- | 2346-81-8 |
| phenol, 2-chloro-4-(1,1-dimethylpropyl)- | 98-28-2 |
| tribromomethane | 75-25-2 |
| 1H-pyrrole | 109-97-7 |
| 1H-pyrrole, 1-methyl- | 96-54-8 |
| 2-furfural | 98-01-1 |
| furan, 2-acetyl- | 1192-62-7 |
| furan, 2-ethyl- | 3208-16-0 |
| furan, 2-pentyl- | 3777-69-3 |
| furan, 3-methyl | 930-27-8 |
| furan, 5-methyl-2-propionyl- | 33978-70-0 |
| pyrazine | 290-37-9 |
| pyrazine, 2,5-dimethyl- | 123-32-0 |
| pyrazine, ethyl- | 13925-00-3 |

(continued)

| Molecule name | CAS number |
|---|---|
| pyrazine, methyl- | 109-08-0 |
| pyridine | 110-86-1 |
| 2-butenenitrile | 4786-20-3 |
| 2-piperidinone | 675-20-7 |
| 3-butenenitrile | 109-75-1 |
| benzene isocyanato | 103-71-9 |
| benzonitrile | 100-47-0 |
| butanenitrile, 3-methyl- | 625-28-5 |
| ethane, isocyano- | 624-79-3 |
| ethyl isocyanate | 109-90-0 |
| propanenitrile | 107-12-0 |
| propanenitrile, 2-methyl- | 78-82-0 |
| 1-hexanol | 111-27-3 |
| 1-hexanol, 2-ethyl- | 104-76-7 |
| 1-pentanol | 71-41-0 |
| 1-propanol, 2-methyl- | 78-83-1 |
| 2-butanol | 15892-23-6 |
| 2-butanol, 2-methyl- | 75-85-4 |
| 2-hexanol, 2,5-dimethyl- | 3730-60-7 |
| 2-pentanol, 2-methyl- | 590-36-3 |
| 2-propanol, 2-methyl- | 75-65-0 |
| 3-hexanol | 623-37-0 |
| ethanol | 64-17-5 |
| phenol | 108-95-2 |
| phenol, 4-(1,1-dimethylpropyl)- | 80-46-6 |
| skatole | 83-34-1 |
| 1,3-isobenzofuranedione | 85-44-9 |
| 2,3-octanedione | 585-25-1 |
| 2,4-pentanedione, 3-methyl- | 815-57-6 |
| 2,6-di-tert-butyl-p-benzoquinone | 719-22-2 |
| 2-butanone | 78-93-3 |
| 2-butanone, 3,3-dimethyl- | 75-97-8 |
| 2-butanone, 3-hydroxy- | 513-86-0 |
| 2-cyclohexen-1-one, 3,4,4-trimethyl- | 17299-41-1 |
| 2-cyclopenten-I-one, 2,3-dimethyl- | 1121-05-7 |
| 2-cyclopenten-1-one, 3-methyl- | 2758-18-1 |
| 2-hexanone, 3-methyl- | 2550-21-2 |
| 2-methyl-2-cyclopenten-1-one | 1120-73-6 |

(continued)

| Molecule name | CAS number |
|---|---|
| 3-heptanone | 106-35-4 |
| 3-hepten-2-one, 5-methyl- | 5090-16-4 |
| 3-octanone | 106-68-3 |
| 3-penten-2-one, 3-methyl- | 565-62-8 |
| 5-hepten-2-one, 6-methyl- | 110-93-0 |
| acetophenone | 98-86-2 |
| cyclohexanone | 108-94-1 |
| cyclopentanone, 2-methyl- | 1120-72-5 |
| cyclopentanone, 3-methyl- | 1757-42-2 |
| methylbutanone | 563-80-4 |
| p-methylacetophenone | 122-00-9 |
| disulfide, dimethyl | 624-92-0 |
| methanethiol | 74-93-1 |
| sulfone, dimethyl- | 67-71-0 |
| dimethyl sulfide | 75-18-3 |
| (Z)-caryophyllene | 118-45-0 |
| anethole (E) | 4180-23-8 |
| a-pinene | 80-56-8 |
| a-terpineol | 10482-56-1 |
| a-thujene | 2867-05-2 |
| b-bourbonene | 5208-59-3 |
| b-caryophyllene | 87-44-5 |
| b-pinene | 127-91-3 |
| camphene | 79-92-5 |
| carvone | 2244-16-8 |
| cis-p-menthan-3-one | 491-07-6 |
| dihydromyrcenol | 18479-59-9 |
| eucalyptol | 470-82-6 |
| limonene | 138-86-3 |
| m-mentha-6,8-diene | 1461-27-4 |
| neo-menthol | 491-01-0 |
| piperitone | 89-81-6 |
| p-menth-3-ene | 500-00-5 |
| trans-p-menthan-3-one<br>benzaldehyde | 89-80-5<br>100-52-7 |
| 2-octanone | 111-13-7 |
| 2-heptanone | 110-43-0 |
| 2,3-pentanedione | 600-14-6 |

(continued)

| Molecule name | CAS number |
|---|---|
| 2-pentanone, 3-methyl- | 565-61-7 |
| acetic acid | 64-19-7 |
| 3-hexanone | 589-38-8 |
| 1-propene, 2-methyl- | 115-11-7 |
| benzaldehyde, 2-methyl- | 529-20-4 |
| propanoic acid | 79-09-4 |
| butanoic acid | 107-92-7 |
| 3-pentanone, 2-methyl- | 565-69-5 |
| 2-pentanone 4,4-dimethyl- | 590-50-1 |
| ethyl acetate | 141-78-6 |
| thiophene, 3-methyl- | 616-44-4 |
| 2-pentanone | 107-87-9 |
| 2-hexanone | 591-78-6 |
| isovaleric acid | 503-74-2 |
| 2-pentanone,4-methyl- | 108-10-1 |
| 2-butene, 2-methyl- | 513-35-9 |
| 1-propanol | 71-23-8 |
| 2-butenal, 2-methyl (E) | 497-03-0 |
| butanoic acid, 2-methyl- | 116-53-0 |
| 2-propanol | 67-63-0 |
| isobutyric acid | 79-31-2 |
| benzene, 2-methyl-1-propenyl- | 768-49-0 |
| hexanoic acid | 142-62-1 |
| acetone | 67-64-1 |
| pentanoic acid, 4-methyl- | 646-07-1 |
| cyclopropane, 1,2-dimethyl (cis) | 930-18-7 |
| p-cresol | 106-44-5 |
| 2,3-butanedione | 431-03-8 |
| 1-butanol, 3-methyl- | 123-51-3 |
| m-methyl acetophenone | 585-74-0 |
| 3-hexen-2-one, 5-methyl- | 5166-53-0 |
| indole | 120-72-9 |
| 3-hexen-2-one, 3,4-dimethyl- | 1635-02-5 |
| pentanal | 110-62-3 |
| 2-pentanol, 2,3-dimethyl- | 4911-70-0 |
| m-cymene | 535-77-3 |
| cyclohexanone | 108-94-1 |
| aniline | 62-53-3 |

(continued)

| Molecule name | CAS number |
|---|---|
| furan, 2-methyl- | 534-22-5 |
| 3-pentanone, 2,4 dimethyl- | 565-80-0 |
| 3-buten-2-one 3-methyl | 814-78-8 |
| hexanenitrile | 628-73-9 |
| heptanenitrile | 629-08-3 |
| pentane nitrile | 110-59-8 |
| butanenitrile | 109-74-0 |
| acrylonitrile | 107-13-1 |

From our experimental studies, 192 volatile molecules have been identified in the volatile fraction of human saliva (table 2). Principal volatile compounds identified in saliva are ketones, acids, aldehydes, alcohols and aromatic compounds.

Among these compounds, 57 volatile compounds have been preselected to be used as possible biomarkers discriminating factor for oral cancer early detection (table 3).

**Table 3: Volatile compounds potentially indicative of oral cancer susceptibility**

| Molecule name | CAS Number |
|---|---|
| benzaldehyde | 100-52-7 |
| 2-octanone | 111-13-7 |
| 2-heptanone | 110-43-0 |
| 2,3-pentanedione | 600-14-6 |
| 3-methyl-2-pentanone | 565-61-7 |
| acetic acid | 64-19-7 |
| 3-hexanone | 589-38-8 |
| 2-methyl-1-propene | 115-11-7 |
| 2-methyl-benzaldehyde | 529-20-4 |
| propanoic acid | 79-09-4 |
| butanoic acid | 107-92-7 |
| 2-methyl-3-pentanone | 565-69-5 |
| 4,4-dimethyl-2-pentanone | 590-50-1 |
| ethyl acetate | 141-78-6 |
| 3-methyl-thiophene | 616-44-4 |
| 2-pentanone | 107-87-9 |
| 2-hexanone | 591-78-6 |
| isovaleric acid | 503-74-2 |
| 4-methyl-2-pentanone | 108-10-1 |
| 2-methyl-2-butene | 513-35-9 |
| 1-propanol | 71-23-8 |
| (E) 2-methyl-2-butenal | 497-03-0 |
| 2-methyl-butanoic acid | 116-53-0 |

(continued)

| Molecule name | CAS Number |
|---|---|
| 2-propanol | 67-63-0 |
| isobutyric acid | 79-31-2 |
| 2-methyl-1-propenyl-benzene | 768-49-0 |
| hexanoic acid | 142-62-1 |
| acetone | 67-64-1 |
| 4-methyl-pentanoic acid | 646-07-1 |
| (cis) 1,2-dimethyl cyclopropane | 930-18-7 |
| p-cresol | 106-44-5 |
| 2,3-butanedione | 431-03-8 |
| 3-methyl-1-butanol | 123-51-3 |
| m-methyl acetophenone | 585-74-0 |
| 5-methyl-3-hexen-2-one | 5166-53-0 |
| indole | 120-72-9 |
| 3,4-dimethyl-3-hexen-2-one | 1635-02-5 |
| pentanal | 110-62-3 |
| 2,3-dimethyl-2-pentanol | 4911-70-0 |
| m-cymene | 535-77-3 |
| cyclohexanone | 108-94-1 |
| aniline | 62-53-3 |
| 2-methyl-furan | 534-22-5 |
| 2,4 dimethyl-3-pentanone | 565-80-0 |
| 3-methyl-3-buten-2-one | 814-78-8 |
| hexanenitrile | 628-73-9 |
| heptanenitrile | 629-08-3 |
| pentane nitrile | 110-59-8 |
| butanenitrile | 109-74-0 |
| 3-methyl-2-pentanone | 565-61-7 |
| methylbutanone | 563-80-4 |
| butanal | 123-72-8 |
| hexanal | 66-25-1 |
| phenol | 108-95-2 |
| m-xylene | 108-38-3 |
| p-xylene | 106-42-3 |
| ethanal | 75-07-0 |
| benzene | 71-43-2 |
| acrylonitrile | 107-13-1 |

According to our results, at least 19 of these 57 compounds are indeed correlated with oral cancer, as shown below.

**Example 8: Quantification of the identified volatile compounds in cancer/healthy patients**

The quantification of the volatile compounds is made by comparison with standard controls that have been added in the preservation buffer at the beginning of the experiment. In this case, the followings molecular standards have been used:

- 1-bromobutane (CAS number 109-65-9)
- 1-bromobenzene (CAS number 108-86-1)
- 1,4-dibromobenzene (CAS number 106-37-6)

**Table 4: Particular biomarkers indicative of oral cancer predisposition**

| Biomarker name | Overexpression in oral cancer population | Overexpression in normal population |
|---|---|---|
| 2,3-pentanedione | 6.00 X | |
| 3 -methyltiophene | 1.50 X | |
| acetone | 2.30 X | |
| hexanitrile | 3.00X | |
| benzaldehyde | 1.80 X | |
| 3-methyl-2-pentanone | 2.10 X | |
| 2,3-butanedione | 4.40 X | |
| 2-propanol | 2.80 X | |
| ethyl acetate | 3.90 X | |
| 1-propanol | 1.90 X | |
| hexanal | 1.60 X | |
| 5-methyl-3-hexen-2-one | 1.70 X | |
| m- and p-xylene | 1.50 X | |
| 2-methyl-2-butenal (E) | 1.90 X | |
| 2-methyl-2-butene | | 2.00 X |
| 2-methyl-1-propene | | 3.30 X |
| (cis) 1,2-dimethyl cyclopropane | | 1.70 X |

It has been concluded from these data that the "detection" of a certain volatile compound in a saliva sample of a patient means that said sample contains at least 1,5 fold the amount of said compound in the normal population.

**Example 9: Statistical analysis of the presence of biochemical organic compounds in the saliva of oral cancer patient vs healthy individuals**

Software STATISTICA version 8.0 of StatSoft France (2007) is used for data analysis. The significances of the differences between the groups were tested by from Factorial Discriminating Analysis (FDA). Thus the similarities or the differences of the samples can be visualized graphically.

The identification of the volatile molecules is obtained by:

- Comparison of the experimental indices of retention to those of the internal data bank,
- comparison of the experimental spectra to those of the bank Wiley 275K and NIST 2.0d, built april 2005.

**- Statistical model 1**

Total population tested is 45 human subjects from two distinctive environmental geographic areas. Oral cancer population is confirmed by visual diagnostics performed by an anticancer center.

The statistical analyzes were carried out on 109 volatile compounds. Abundances of the molecules in each sample were reported to abundances of the 3 internal standards analyzed with saliva. The principal volatile compounds identified in saliva are ketones, acids, aldehydes, alcohols and aromatic compounds. All the samples have a strong abundance in hydrazoic acid coming directly from the buffer solution of conservation.

On the 108 volatile compounds, 49 are significant to separate the group "tumor" from the reference group. A discriminating factorial analysis on these variables makes it possible to classify well 97.78% of the samples with 4 volatile compounds: the hexanenitrile, the 2, 3- pentanedione, 3- methylthiophene and acetone. Only 1 false- positive have been detected with the statistical model 1.

**- Statistical model 2**

Total population tested is 45 human subjects from two distinctive environmental geographic areas. Oral cancer population is confirmed by visual diagnostics performed by a specialized anticancer center.

The statistical analyzes were carried out on 108 volatile compounds. Abundances of the molecules in each sample were reported to abundances of the 3 internal standards analyzed with saliva. The principal volatile compounds identified in saliva are ketones, acids, aldehydes, alcohols and aromatic compounds. All the samples have a strong abundance in hydrazoic acid coming directly from the buffer solution of conservation.

All nitriles volatile compounds have been removed from the statistical model number 2. For this study, the 10 made up "nitriles" were not taken into account.

From the 98 remaining volatile compounds, an ANOVA test according to the factor "tumor" showed that 45 components are significant to separate the group "tumor" from the reference group.

A discriminating factorial analysis on these variables makes it possible to classify well 93, 33% of the samples with 4 volatile compounds: benzaldéhyde, acetone, the 2, 3- pentanedione and 2- methyl- 2- butene. The first 3 molecules are side of the group "tumor" and the 2- methyl- 2- butene on the side of the control group. 3 false- negatives and no false positive have been detected with the statistical model 2.

To conclude, this study highlights the tight link existing between 14 organic compounds (namely hexanenitrile, the 2, 3- pentanedione, 3- methylthiophene, 2- methyl- 2- butylene, 3- methyl- 2- pentanone, 2, 3- butanedione, 2- propanol, ethyl acetate, 1- propanol, hexanal, 5- methyl- 3- hexen- 2- one, m- xylene, p- xylene, 2- methyl- 2- butenal (E) ) and oral cancer in human. It is noteworthy that none of them have ever been found in exhaled breath (Mashir A, Advanced Powder Technology, 2009) or being associated to oral cancer.

**<u>Example 10: Diagnostic test based on the ratios of specific organic molecules</u>**

Software STATISTICA version 8.0 of StatSoft France (2007) is used for data analysis. The significances of the differences between the groups were tested by Factorial Discriminating Analysis (FDA). Thus the similarities or the differences of the samples can be visualized graphically.

Tested population

Total population tested is 52 human subjects from two distinctive environmental geographic areas. Oral cancer population is confirmed by visual diagnostics performed by a specialized anticancer center.

The following volatile organic compounds are used in the diagnostic test:

3-methyl- 2-pentanone (CAS number : 565-61-7)
Methylbutanone (CAS number : 563-80-4)
2.4- dimethyl- 3- pentanone (CAS number: 565- 80- 0)
Benzene (CAS number: 71-43-2)
Phenol (CAS number : 108-95-2)
2.3-butanedione (CAS number : 431-03-8)
5- methyl- 3- hexen- 2- one (CAS number: 5166- 53- 0)
2-methyl- 1-propene (CAS number: 115-11-7)
Butanal (CAS number: 123-72-8)
Hexanal (CAS number : 66-25-1)
2-propanol (CAS number: 67-63-0)

Ethyl acetate (CAS number: 141-78-6)
Hexanenitrile (CAS number : 628-73-9)
1-propanol (CAS number: 71-23-8)
(cis) 1, 2- dimethyl- cyclopropane (CAS number: 930- 18- 7)
m- and p-xylene (CAS number : 108-38-3 and CAS number: 106-42-3)
(E) 2- methyl- 2- butenal (CAS number: 497- 03- 0)
3-methyl-thiophene (CAS number: 616-44-4)
Ethanal (CAS number: 75-07-0)

The median values by group were calculated for the following ratios:

- 3- methyl- 2- pentanone / methyl butanone
- 2.4- dimethyl- 3- pentanone / benzene
- phenol /2,3-butanedione
- 5- methyl- 3- hexen- 2- one / 2- methyl- 1- propene
- butanal / hexanal
- 2-propanol / ethyl acetate
- hexanenitrile / 1-propanol
- 2-propanol / (cis) 1.2-dimethyl cyclopropane
- m- xylene / 2- methyl- 2- butenal
- 3- methyl- 2- pentanone / 3- methyl- thiophene,
- 2,3-butanedione / ethanal

The statistical method used is FDA (Factorial Discrimination Analysis).

The median values by group were calculated for each of the ratios.

The 5 followings were found to be statistically significative of oral cancer patient group or control group:

1) 3- methyl- 2- pentanone / methyl butanone (R1)

2) Butanal / hexanal (R56)

3) Hexanenitrile / 1-propanol (R260)

4) 2-propanol / (cis) 1,2 dimethyl cyclopropane (R266)

5) Phenol / 2,3 butanedione (R269).

Among these ratios, 2 were found to be reproducibly correlated with healthy subjects, and three were indicative of oral cancer suffering patients (table 5).

**Table 5: Median values for the 5 ratios correlated with oral cancer or healthiness**

| | R1 | R56 | R260 | R266 | R269 |
|---|---|---|---|---|---|
| | 3-methyl-2-pentanone / methyl butanone | Butanal / hexanal | Hexanenitrile / 1-propanol | 2-propanol / (cis) 1,2-dimethyl cyclopropane | Phenol / 2,3-butanedione |
| **Average healthy** | 0,182 | 0,223 | 0,040 | 2,939 | 0,696 |
| **Average Oral Cancer** | 0,357 | 0,138 | 0,089 | 9,359 | 0,217 |

The 3 ratios R1 (3- methyl- 2- pentanone / methyl butanone), R260 (Hexanenitrile / 1- propanol) and R266 (Phenol / 2, 3- butanedione) are of the side of the oral cancer group group and the values are respectively 1, 96 ; 2, 24 and 3, 18 times higher in this group than in the healthy group.

The 2 ratios R56 (Butanal / hexanal) and R269 (Phenol / 2, 3- butanedione) are of the side of the healthy group and are respectively 1, 62 and 3, 21 higher in this group than in the oral cancer group.

The absolute limiting values of the ratios permitting to classify the patients in a potential "oral cancer group" are given in table 6:

Table 6: Absolute limiting ratio values permitting to **classify** the patients

| **Report/ratio** | **Condition so that the sample is "oral cancer risk"** | **Nb samples corresponding to the ratio** |
|---|---|---|
| 3-methyl-2-pentanone / methyl butanone | > 0.344 | 17 |
| Butanal / hexanal | < 0.11 | 10 |
| Hexanenitrile / 1-propanol | > 0.167 | 3 |
| 2-propanol / (cis) 1,2-dimethyl cyclopropane | > 10.33 | 9 |
| Phenol / 2,3-butanedione | < 0.005 | 4 |

To classify the samples it is necessary to apply the following formula (from the FDA statistical method) taking into account all these 5 ratios (linear combination of the 5 variable ratios):

$$\text{Factor } X = 1.8277 - 7.3472*R1 - 0.125*R266 - 14.2293*R260 + 1.2050*R269 + 8.883*R56$$

If factor **X≤0.6,** the sample is classified in the Oral Cancer Risk Population
If factor **X>0.6,** the sample is classified healthy

Therefore, the method of the invention, based on:

i) the recovery of the volatile fraction of the saliva of a human subject,

i) the quantification of ten biochemical organic compounds (3- methyl- 2- pentanone, methyl butanone, Butanal, hexanal, Hexanenitrile, 1- propanol 2- propanol, (cis) 1, 2- dimethyl cyclopropane, phenol, and 2, 3- butanedione) in said volatile fraction,

ii) calculation of the ratios R1, R266, R260, R269 and R56 as mentioned above,

iii) calculation of said factor X and its comparison with the threshold 0,6,

enables the man skilled in the art to prognose and/or diagnose an oral cancer in said human subject.

The analysis of the ratios between the organic compounds: 3- methyl- 2- pentanone / methyl butanone, Butanal / hexanal, Hexanenitrile / 1- propanol 2- propanol / (cis) 1, 2- dimethyl cyclopropane, and Phenol / 2, 3- butanedione in the volatile fraction of the saliva of a human subject permits to obtain a highly sensitive test of predisposition of oral cancer (98.077% sensitivity; 1 false- positive) (figure 1) .

**Example 11: Combination of the biomarkers of fluid fraction and volatile fraction for diagnosing/prognosing oral cancer.**

Software STATISTICA version 8.0 of StatSoft France (2007) is used for data analysis. The significances of the differences between the groups were obtained from Factorial Discriminating Analysis (FDA). Thus the similarities or the differences of the samples can be visualized graphically.

The best combination of biomarker was the following: *Streptococcus mitis* + SSAT + H3F3A + hexanenitrile + 2.3-pentanedione, 3-methylthiophene + acetone. Indeed the use of such markers permits to obtain 100% sensibility results as shown in table 7.

**Table 7: Sensibility of the diagnostic test based on biomarkers from the fluid fraction and volatile fraction**

| | **bacteria** | **human mRNA** | **volatile compounds statistical model 1** | **bacteria + human mRNA** | **bacteria + hmRNA+volatile compounds** |
|---|---|---|---|---|---|
| **Specificity %** | 76 | 54 | 98 | 83 | 98 |
| **Sensibility %** | 79 | 32 | 100 | 89 | 100 |

## Claims

1. An *in vitro* method of diagnosing a predisposition to oral cancer in a human subject or of diagnosing an oral cancer in a human subject, the method comprising collecting and stabilizing a crude saliva sample from said human subject, and performing at least the following step:

    * Analyzing the volatile fraction corresponding to the evaporable part extracted from said stabilized saliva sample by detecting in said volatile fraction at least one biochemical organic compound;

    wherein the detection of at least one biochemical organic compound is indicative of a risk or a predisposition to develop oral cancer.

2. The method of claim 1, wherein said volatile fraction is extracted from crude saliva sample by heating said saliva sample for at least 10 minutes at a temperature comprised between 30°C and 50°C.

3. The method of claim 1, wherein said at least one biochemical organic compound is selected in the group consisting of: 2, 3- pentanedione (CAS number 600- 14- 6), 3- methylthiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3), 2- methyl- 2- butenal (E) (CAS number 497- 03- 0), phenol (CAS number 108- 95- 2), butanal (CAS number 123- 72- 8), methylbutanone (CAS number: 563- 80- 4), 2- methyl- 2- butene (CAS number 513- 35- 9), 2- methyl- 1- propene (CAS number 115- 11- 7) and *(cis)* 1, 2 dimethyl- cyclopropane (CAS number: 930- 18- 7) ; and preferably the detection of at least one biochemical organic compound chosen among 2, 3- pentanedione (CAS number 600- 14- 6), 3- methyltiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3), 2- methyl- 2- butenal (E) (CAS number 497- 03- 0) in the volatile fraction of saliva of an human subject indicates that said human subject has a high risk of developing an oral cancer.

4. The method according to claim 3, wherein the detection of the biochemical organic compounds of the group comprising: hexanenitrile, 2, 3- pentanedione, 3- methylthiophene and acetone in the volatile fraction of saliva of a human subject indicates that said human subject is developing an oral cancer.

5. The method according to claim 3, wherein the detection of at least one biochemical organic compound chosen in the group comprising: 2- methyl- 2- butene (CAS number 513- 35- 9), 2- methyl- 1- propene (CAS number 115- 11-7) and (*cis*) 1, 2- dimethyl cyclopropane (CAS number 930- 18- 7) in the volatile fraction of saliva of a human subject indicates that said human subject is not developing an oral cancer.

**6.** The method of claim 1, wherein said method further comprises the step of:

* analyzing a fluid fraction of said stabilized saliva sample by detecting specific DNA or RNA sequences of human, bacterial or viral origin in said fluid fraction.

**7.** The method of claim 6, wherein said specific DNA or RNA sequences are chosen among:

i) human sequences selected from SSAT mRNA (SEQ ID No 62), H3F3A mRNA (SEQ ID No 63) and IL8 mRNA (SEQ ID No 64); and/or
ii)sequences of bacteria selected from *Capnocytophaga gingivalis* (ATCC 33624), *Prevotella melaninogenica* (ATCC 25845), *Streptococcus mitis* (ATCC 15914) and *Micrococcus luteus* (ATCC 53598D); and/or
iii) the viral sequences of human papillomavirus, preferably said human papillomavirus is human papillomavirus 16 (ATCC 45113) or human papillomavirus 18 (ATCC 45152);

and preferably wherein the detection of at least two human mRNA sequences as specifically defined in i) and at least one bacterial sequence as specifically defined in ii) in the saliva of a human subject indicates that said human subject has a high risk of developing an oral cancer.

**8.** The method according to claim 7, wherein the detection of the human mRNA of H3F3A (SEQ ID No 63), of the human mRNA of SSAT (SEQ ID No 62) and of the bacterial genome of *Streptococcus mitis* (ATCC 15914) in the saliva of a human subject indicates that said human subject has a high of developing an oral cancer.

**9.** The method according to claim 7, wherein:

i) the crude saliva is stabilized by using a solution comprising a salt such as guanidium thiocyanate, and/or ammonium sulfate, and/or sodium azide, and optionally exo and/or endonuclease inhibitors; and/or
ii) specific DNA or RNA sequences are detected by incubating said genomic DNA and total RNA with a thermostable enzyme with RNA-dependent Reverse Transcriptase activity and with DNA-dependent Polymerase activity, preferably the combination of RT-PCR and PCR is performed in a single-tube reaction.

**10.** The method according to claim 7, wherein the detection of the mRNA sequence of H3F3A (SEQID No 63), of SSAT (SEQ ID No 62) and the detection of the biochemical organic compounds hexanenitrile, 2, 3- pentanedione, 3-methylthiophene and acetone in the saliva of a human subject indicates that said human subject is developing an oral cancer.

**11.** The method according to claim 1, wherein:

i) the volatile fraction of saliva is extracted from crude saliva sample by heating said saliva sample for at least 10 minutes at 40°C by using a Solid-phase Microextraction (SPME) with a CAR/PDMS fibe; and/or
ii) the biochemical organic compounds in said volatile fraction are detected by using a chromatograph in gas phase coupled to a mass spectrometer.

**12.** The method according to claim 7, comprising amplifying and detecting at least one DNA or RNA sequence chosen in the group comprising: SEQ ID N0 62 to 70.

**13.** The method according to claim 7, comprising the steps of:

a) collecting a sample of crude saliva of said human subject in a sterile device,
b) stabilizing said sample by adding a solution comprising a guanidium salt, such as guanidinum thiocyanate, and/or ammonium sulfate, and/or sodium azide, and optionally exo and/or endonuclease inhibitors,
c) extracting total nucleic acid of bacteria, virus, and human origins from the previously obtained stabilized saliva sample,
d) precipitation and purification of total nucleic acids,
e) incubating the purified total nucleic acid with a thermostable enzyme with RNA-dependant reverse transcriptase activity and with DNA-dependant polymerase activity and polynucleotide primers under conditions which allow the reverse transcriptase activity of said thermostable enzyme to synthetise cDNA from the ribonucleic and amplification of genomic DNA and cDNA at a detectable level by Polymerase Chain Reaction,
f) detecting in an assay the amplified DNAs sequences by hybridization with one or more polynucleotide probes specific to :

i) human sequences selected from SSAT mRNA (SEQ ID No 62), H3F3A mRNA (SEQ ID No 63) and IL8 mRNA (SEQ ID No 64); or
ii) sequences of bacteria selected from Capnocytophaga gingivalis (ATCC 33624, SEQ ID No 65), Prevotella melaninogenica (ATCC 25845, SEQ ID No 66), Streptococcus mitis (ATCC 15914, SEQ ID No 67) and Micrococcus luteus (ATCC 53598D, SEQ ID No 68), or
iii) sequences of virus selected from human papillomavirus 16 (ATCC 45113, SEQ ID No 69) and human papillomavirus 18 (ATCC 45152, SEQ ID No 70);

and wherein said primers and said probes used to amplify and detect at least one sequence of i), ii) or iii) are preferably selected from the group consisting of SEQ ID No 1 to SEQ ID No 60.

**14.** The method according to claim 1, comprising the steps of:

a) collecting a sample of crude saliva of said human subject in a sterile device,
b) stabilizing said sample by adding a solution comprising a guanidium salt, such as guanidinum thiocyanate, ammonium sulfate and/or sodium azide,
c) extracting the volatile fraction from said stabilized sample by heating it for at least 10 minutes at 40°C and using for example solid-phase microextraction (SPME) to take away the volatile fraction,
d) detecting at least one biochemical organic compound by using for example a chromatograph in gas phase coupled to a mass spectrometer, wherein said at least one biochemical organic compounds is preferably chosen among: 2, 3- pentanedione (CAS number 600- 14- 6), 3- methylthiophene (CAS number 616- 44- 4), acetone (CAS number 67- 64- 1), hexanenitrile (CAS number 628- 73- 9), benzaldehyde (CAS number 100- 52- 7), 3- methyl- 2- pentanone (CAS number 565- 61- 7), 2, 3- butanedione (CAS number 431- 03- 8), 2- propanol (CAS number 67- 63- 0), ethyl acetate (CAS number 141- 78- 6), 1- propanol (CAS number 71- 23- 8), hexanal (CAS number 66- 25- 1), 5- methyl- 3- hexen- 2- one (CAS number 5166- 53- 0), m- xylene (CAS number 108- 38- 3), p- xylene (CAS number 106- 42- 3), 2- methyl- 2- butenal (E) (CAS number 497- 03- 0), phenol (CAS number 108- 95- 2), butanal (CAS number 123- 72- 8), methylbutanone (CAS number: 563- 80- 4), 2- methyl- 2- butene (CAS number 513- 35- 9), 2- methyl- 1- propene (CAS number 115- 11- 7) and *(cis)* 1, 2 dimethyl- cyclopropane (CAS number: 930- 18- 7) .

**15.** Use for practicing the method according to claim 14 of a kit comprising:

a) A sterile device to collect a saliva sample, optionally containing a collect reagent
b) A preservation reagent,
c) At least one electronic sensor,
d) Optionally, a control molecular marker.

**Patentansprüche**

**1.** In vitro-Verfahren zur Diagnose einer Prädisposition für Mundhöhlenkarzinome bei einem Menschen oder zur Diagnose eines Mundhöhlenkarzinoms bei einem Menschen, wobei das Verfahren die Entnahme und Stabilisierung einer unbehandelten Speichelprobe von dem betreffenden Menschen und die Durchführung mindestens des folgenden Schrittes umfasst:

* Untersuchen der flüchtigen Fraktion, welche dem Anteil entspricht, der aus der stabilisierten Speichelprobe durch Verdampfen genommen werden kann, indem in der flüchtigen Fraktion mindestens eine biochemische organische Verbindung detektiert wird;

wobei der Nachweis mindestens einer biochemischen organischen Verbindung ein Risiko oder eine Prädisposition für die Entwicklung von Mundhöhlenkarzinomen anzeigt.

**2.** Verfahren nach Anspruch 1, wobei die flüchtige Fraktion aus der unbehandelten Speichelprobe gewonnen wird, indem die Speichelprobe mindestens 10 Minuten lang auf eine Temperatur im Bereich von 30 °C bis 50 °C erwärmt wird.

**3.** Verfahren nach Anspruch 1, wobei die mindestens eine biochemische organische Verbindung aus der Gruppe ausgewählt ist, die aus den folgenden besteht: 2, 3- Pentandion (CAS- Nummer 600- 14- 6), 3- Methylthiophen (CAS- Nummer 616- 44- 4), Aceton (CAS- Nummer 67- 64- 1), Hexannitril (CAS- Nummer 628- 73- 9), Benzaldehyd (CAS- Nummer 100- 52- 7), 3- Methyl- 2- pentanon (CAS- Nummer 565- 61- 7), 2, 3- Butandion (CAS- Nummer 431- 03- 8), 2- Propanol (CAS- Nummer 67- 63- 0), Ethylacetat (CAS- Nummer 141- 78- 6), 1- Propanol (CAS- Nummer 71- 23- 8), Hexanal (CAS- Nummer 66- 25- 1), 5- Methyl- 3- hexen- 2- on (CAS- Nummer 5166- 53- 0), m- Xylol (CAS- Nummer 108- 38- 3), p- Xylol (CAS- Nummer 106- 42- 3), 2- Methyl- 2- butenal (E) (CAS- Nummer 497- 03- 0), Phenol (CAS- Nummer 108- 95- 2), Butanal (CAS- Nummer 123- 72- 8), Methylbutanon (CAS- Nummer: 563- 80- 4), 2- Methyl- 2- buten (CAS- Nummer 513- 35- 9), 2- Methyl- 1- propen (CAS- Nummer 115- 11- 7) und *(cis)* 1, 2 Dimethylcyclopropan (CAS- Nummer: 930- 18- 7) ; und wobei vorzugsweise der Nachweis von mindestens einer biochemischen organischen Verbindung, die aus 2, 3- Pentandion (CAS- Nummer 600- 14- 6), 3- Methyltiophen (CAS- Nummer 616- 44- 4), Aceton (CAS- Nummer 67- 64- 1), Hexannitril (CAS- Nummer 628- 73- 9), Benzaldehyd (CAS- Nummer 100- 52- 7), 3- Methyl- 2- pentanon (CAS- Nummer 565- 61- 7), 2, 3- Butandion (CAS- Nummer 431- 03- 8), 2- Propanol (CAS- Nummer 67- 63- 0), Ethylacetat (CAS- Nummer 141- 78- 6), 1- Propanol (CAS- Nummer 71- 23- 8), Hexanal (CAS- Nummer 66- 25- 1), 5- Methyl- 3- hexen- 2- on (CAS- Nummer 5166- 53- 0), m- Xylol (CAS- Nummer 108- 38- 3), p- Xylol (CAS- Nummer 106- 42- 3), 2- Methyl- 2- butenal (E) (CAS- Nummer 497- 03- 0) in der flüchtigen Fraktion des Speichels eines Menschen anzeigt, dass bei diesem Menschen ein hohes Risiko für die Entwicklung eines Mundhöhlenkarzinoms besteht.

**4.** Verfahren nach Anspruch 3, wobei der Nachweis der biochemischen organischen Verbindungen aus der Gruppe, die folgendes umfasst: Hexannitril, 2, 3- Pentandion, 3- Methylthiophen und Aceton in der flüchtigen Fraktion des Speichels eines Menschen anzeigt, dass sich bei dem Menschen ein Mundhöhlenkarzinom entwickelt.

**5.** Verfahren nach Anspruch 6, wobei der Nachweis von mindestens einer biochemischen organischen Verbindung, welche aus der Gruppe ausgewählt ist, die Folgendes umfasst: 2- Methyl- 2- buten (CAS- Nummer 513- 35- 9), 2- Methyl- 1- propen (CAS- Nummer 115- 11- 7) und (cis) 1, 2- Dimethylcyclopropan (CAS- Nummer 930- 18- 7) in der flüchtigen Fraktion des Speichels eines Menschen anzeigt, dass sich bei diesem Menschen kein Mundhöhlenkarzinom entwickelt.

**6.** Verfahren nach Anspruch 1, wobei das Verfahren weiterhin den folgenden Schritt umfasst:

* Untersuchen einer fließfähigen Fraktion der stabilisierten Speichelprobe, indem in der fließfähigen Fraktion spezifische DNA- oder RNA-Sequenzen menschlichen, bakteriellen oder viralen Ursprungs nachgewiesen werden.

**7.** Verfahren nach Anspruch 6, wobei die spezifischen DNA- oder RNA-Sequenzen aus den folgenden ausgewählt sind:

i) menschlichen Sequenzen, die aus SSAT mRNA (SEQ ID Nr. 62), H3F3A mRNA (SEQ ID Nr. 63) und IL8 mRNA (SEQ ID Nr. 64) ausgewählt sind; und/oder
ii)Sequenzen von Bakterien, die aus *Capnocytophaga gingivalis* (ATCC 33624), *Prevotella melaninogenica* (ATCC 25845), *Streptococcus mitis* (ATCC 15914) und *Micrococcus luteus* (ATCC 53598D) ausgewählt sind; und/oder
iii) den viralen Sequenzen des menschlichen Papillomavirus, wobei es sich bei dem menschlichen Papillomavirus vorzugsweise um das menschliche Papillomavirus 16 (ATCC 45113) oder das menschliche Papillomavirus 18 (ATCC 45152) handelt;

und wobei vorzugsweise der Nachweis von mindestens zwei menschlichen mRNA-Sequenzen gemäß den Angaben unter i) und mindestens einer bakteriellen Sequenz gemäß den Angaben unter ii) im Speichel eines Menschen anzeigt, dass bei diesem Menschen ein hohes Risiko für die Entwicklung eines Mundhöhlenkarzinoms besteht.

**8.** Verfahren nach Anspruch 7, wobei der Nachweis der menschlichen mRNA von H3F3A (SEQ ID Nr. 63), der menschlichen mRNA von SSAT (SEQ ID Nr. 62) und des bakteriellen Genoms von *Streptococcus mitis* (ATCC 15914) im Speichel eines Menschen anzeigt, dass bei diesem Menschen ein hohes Risiko für die Entwicklung eines Mundhöhlenkarzinoms besteht.

**9.** Verfahren nach Anspruch 7, wobei:

i) der unbehandelte Speichel durch die Verwendung einer Lösung stabilisiert wird, die ein Salz wie etwa Guadiniumthiocyanat und/oder Ammoniumsulfat und/oder Natriumazid sowie möglicherweise Exo- und/oder Endonukleasehemmstoffe umfasst; und/oder
ii) spezifische DNA- oder RNA-Sequenzen nachgewiesen werden, indem die genomische DNA und die Gesamt-RNA mit einem wärmestabilen Enzym inkubiert werden, das eine RNA-abhängige reverse-Transkriptase-Aktivität und eine DNA-abhängige Polymerase-Aktivität aufweist, wobei die Kombination aus RT-PCR und PCR vorzugsweise in ein- und demselben Reaktionsgefäß durchgeführt wird.

**10.** Verfahren nach Anspruch 7, wobei der Nachweis der mRNA- Sequenz von H3F3A (SEQ ID Nr. 63), von SSAT (SEQ ID Nr. 62) und der Nachweis der biochemischen organischen Verbindungen Hexannitril, 2, 3- Pentandion, 3-Methylpentandion und Aceton im Speichel eines Menschen anzeigen, dass sich bei diesem Menschen ein Mundhöhlenkarzinoms entwickelt.

**11.** Verfahren nach Anspruch 1, wobei

i) die flüchtige Fraktion des Speichels aus der unbehandelten Speichelprobe gewonnen wird, indem die Speichelprobe mindestens 10 Minuten lang auf 40 °C erwärmt wird, wobei eine Festphasenmikroextraktion (SPME) mit einer CAR/PDMS-Faser erfolgt; und/oder
ii) die biochemischen organischen Verbindungen in der flüchtigen Fraktion nachgewiesen werden, indem ein Gaschromatograph verwendet wird, der mit einem Massenspektrometer gekoppelt ist.

**12.** Verfahren nach Anspruch 7, welches die Vervielfältigung und den Nachweis mindestens einer DNA- oder RNA-Sequenz umfasst, die aus der Gruppe ausgewählt ist, welche Folgendes umfasst: SEQ ID Nr. 62 bis 70.

**13.** Verfahren nach Anspruch 7, welches die folgenden Schritte umfasst:

a) Überführen einer Probe unbehandelten Speichels eines Menschen in eine sterile Vorrichtung,
b) Stabilisieren der Probe durch Zusatz einer Lösung, die ein Guadiniumsalz, wie etwa Guadiniumthiocyanat, und/oder Ammoniumsulfat und/oder Natriumazid sowie möglicherweise Exo- und/oder Endonukleasehemmstoffe umfasst,
c) Gewinnen der Gesamtnukleinsäure von Bakterien, Viren sowie menschlichen Ursprungs aus der zuvor erhaltenen stabilisierten Speichelprobe,
d) Fällen und Aufreinigen der Gesamtmenge an Nukleinsäuren,
e) Inkubieren der aufgereinigten Gesamtnukleinsäure mit einem wärmestabilen Enzym, das eine RNA-abhängige reverse-Transkriptase-Aktivität und eine DNAabhängige Polymerase-Aktivität aufweist, sowie mit Polynukleotidprimern unter Bedingungen, die es ermöglichen, dass die reverse-Transkriptase-Aktivität des wärmestabilen Enzyms zur Synthese von cDNA ausgehend von der Ribonukleinverbindung führt, und Vervielfältigen der genomischen DNA und der cDNA bis zum Erreichen einer Menge, die mittels Polymerase-Kettenreaktion nachgewiesen werden kann.
f) Nachweisen der vervielfältigten DNA-Sequenzen in einem Assay, wobei eine Hybridisierung mit einer oder mehreren Polynukleotidsonden erfolgt, welche für Folgendes spezifisch sind:

i) menschliche Sequenzen, die aus SSAT mRNA (SEQ ID Nr. 62), H3F3A mRNA (SEQ ID Nr. 63) und IL8 mRNA (SEQ ID Nr. 64) ausgewählt sind; oder
ii) Sequenzen von Bakterien, die aus Capnocytophaga gingivalis (ATCC 33624, SEQ ID Nr. 65), Prevotella melaninogenica (ATCC 25845, SEQ ID Nr. 66), Streptococcus mitis (ATCC 15914, SEQ ID Nr. 67) und Micrococcus luteus (ATCC 53598D, SEQ ID Nr. 68) ausgewählt sind, oder
iii) Sequenzen von Viren, die aus dem menschlichen Papillomavirus 16 (ATCC 45113, SEQ ID Nr. 69) und dem menschlichen Papillomavirus 18 (ATCC 45152, SEQ ID Nr. 70) ausgewählt sind;

und wobei die Primer und Sonden, welche dazu verwendet werden, mindestens eine der Sequenzen i), ii) oder iii) zu vervielfältigen und nachzuweisen, vorzugsweise aus der Gruppe ausgewählt sind, die aus SEQ ID Nr. 1 bis SEQ ID Nr. 60 besteht.

**14.** Verfahren nach Anspruch 1, welches die folgenden Schritte umfasst:

a) Überführen einer Probe unbehandelten Speichels eines Menschen in eine sterile Vorrichtung,

b) Stabilisieren der Probe durch Zusatz einer Lösung, die ein Guadiniumsalz, wie etwa Guadiniumthiocyanat, Ammoniumsulfat und/oder Natriumazid umfasst,

c) Gewinnen der flüchtigen Fraktion aus der stabilisierten Probe, indem diese mindestens 10 Minuten lang auf 40 °C erwärmt wir, wobei beispielsweise die Festphasenmikroextraktion (SPME) verwendet wird, um die flüchtige Fraktion zu entnehmen,

d) Nachweisen mindestens einer biochemischen organischen Verbindung, indem beispielsweise ein Gaschromatograph verwendet wird, der mit einem Massenspektrometer gekoppelt ist, wobei die mindestens eine biochemische organische Verbindung vorzugsweise aus den folgenden ausgewählt ist: 2, 3- Pentandion (CAS-Nummer 600- 14- 6), 3- Methylthiophen (CAS- Nummer 616- 44- 4), Aceton (CAS- Nummer 67- 64- 1), Hexannitril (CAS- Nummer 628- 73- 9), Benzaldehyd (CAS- Nummer 100- 52- 7), 3- Methyl- 2- pentanon (CAS- Nummer 565- 61- 7), 2, 3- Butandion (CAS- Nummer 431- 03- 8), 2- Propanol (CAS- Nummer 67- 63- 0), Ethylacetat (CAS- Nummer 141- 78- 6), 1- Propanol (CAS- Nummer 71- 23- 8), Hexanal (CAS- Nummer 66- 25- 1), 5-Methyl- 3- hexen- 2- on (CAS- Nummer 5166- 53- 0), m- Xylol (CAS- Nummer 108- 38- 3), p- Xylol (CAS-Nummer 106- 42- 3), 2- Methyl- 2- butenal (E) (CAS- Nummer 497- 03- 0), Phenol (CAS- Nummer 108- 95- 2), Butanal (CAS- Nummer 123- 72- 8), Methylbutanon (CAS- Nummer: 563- 80- 4), 2- Methyl- 2- buten (CAS-Nummer 513- 35- 9), 2- Methyl- 1- propen (CAS- Nummer 115- 11- 7) und *(cis)* 1, 2 Dimethylcyclopropan (CAS-Nummer: 930- 18- 7) .

**15.** Verwendung eines Kits zur Durchführung des Verfahren nach Anspruch 14, umfassend:

a) eine sterile Vorrichtung zur Entnahme einer Speichelprobe, wobei diese möglicherweise ein Entnahmereagenz enthält

b) ein Konservierungsreagenz,

c) mindestens einen elektronischen Sensor,

d) möglicherweise einen Molekularmarker zur Überprüfung.

**Revendications**

**1.** Une méthode *in vitro* pour le diagnostic d'une prédisposition à un cancer oral chez un sujet humain ou pour le diagnostic d'un cancer oral chez un sujet humain, cette méthode comprenant la collecte et la stabilisation d'un échantillon de salive dudit sujet humain, et la réalisation sur cet échantillon d'au moins les étapes suivantes :

• L'analyse de la fraction volatile correspondant à la partie évaporable extraite dudit échantillon de salive stabilisé par la détection dans la dite fraction volatile d'au moins un composé biochimique organique ;

dans laquelle la détection d'au moins un composé biochimique organique est associé à un risque ou à une prédisposition de développer un cancer oral.

**2.** La méthode selon la revendication 1, dans laquelle ladite fraction volatile est extraite en chauffant cet échantillon de salive à une température comprise entre 30°C et 50°C pendant au moins 10 minutes.

**3.** La méthode selon la revendication 1, dans laquelle le dit composé biochimique organique est issu d'un groupe comprenant : la 2, 3- pentanedione (numéro CAS 600- 14- 6), le 3- méthylthiophène (numéro CAS 616- 44- 4), l'acétone (numéro CAS 67- 64- 1), l'hexanenitrile (numéro CAS 628- 73- 9), le benzaldéhyde (numéro CAS 100-52- 7), la 3- méthyl- 2- pentanone (numéro CAS 565- 61- 7), la 2, 3- butanedione (numéro CAS 431- 03- 8), le 2-propanol (numéro CAS 67- 63- 0), l'acétate d'éthyle (numéro CAS 141- 78- 6), le 1- propanol (numéro CAS 71- 23-8), l'hexanal (numéro CAS 66- 25- 1), la 5- méthyl- 3- hexène- 2- one (numéro CAS 5166- 53- 0), le m- xylène (numéro CAS 108- 38- 3), le p- xylène (numéro CAS 106- 42- 3), le 2- méthyl- 2- butenal (E) (numéro CAS 497-03- 0), le phénol (numéro CAS 108- 95- 2), le butanal (numéro CAS 123- 72- 8), la méthylbutanone (numéro CAS: 563- 80- 4), le 2- méthyl- 2- butène (numéro CAS 513- 35- 9), le 2- méthyl- 1- propène (numéro CAS 115- 11- 7) et le (cis) 1, 2 diméthyl- cyclopropane (numéro CAS: 930- 18- 7) ; et de façon préférée la détection d'au moins un composé biochimique organique choisi parmi la 2, 3- pentanedione (numéro CAS 600- 14- 6), le 3- methyltiophène (numéro CAS 616- 44- 4), l'acétone (numéro CAS 67- 64- 1), l'hexanenitrile (numéro CAS 628- 73- 9), le benzaldéhyde (numéro CAS 100- 52- 7), la 3- méthyl- 2- pentanone (numéro CAS 565- 61- 7), le 2, 3- butanedione (numéro CAS 431- 03- 8), le 2- propanol (numéro CAS 67- 63- 0), l'acétate d'éthyle (numéro CAS 141- 78- 6), le 1- propanol (numéro CAS 71- 23- 8), l'hexanal (numéro CAS 66- 25- 1), la 5- méthyl- 3- Hexen- 2- one (numéro CAS 5166- 53-0), le m- xylène (numéro CAS 108- 38- 3), le p- xylène (numéro CAS 106- 42- 3), la 2- méthyl- 2- butenal (E) (numéro CAS 497- 03- 0) dans la fraction volatile de la salive dudit sujet humain indique que ledit sujet a un risque élevé de développe un cancer oral.

**4.** La méthode selon la revendication 3, dans laquelle la détection des composés biochimiques organiques du groupe comprenant : l'hexanenitrile, la 2, 3- pentanedione, le 3- methylthiophène et l'acétone dans la fraction volatile de la salive d'un sujet humain indique que le dit sujet humain développe un cancer oral.

**5.** La méthode selon la revendication 3, dans laquelle la détection d'au moins un composé biochimique organique choisi dans le groupe comprenant : le 2- méthyl- 2- butène (numéro CAS 513- 35- 9), le 2- méthyl- 1- propène (numéro CAS 115- 11- 7) et le (cis) 1, 2- diméthyl cyclopropane (numéro CAS 930- 18- 7) dans la fraction volatile de la salive d'un sujet humain indique que ledit sujet humain ne développe pas un cancer oral.

**6.** La méthode selon la revendication 1, dans laquelle ladite méthode comprend les étapes suivantes :

• L'analyse de la fraction fluide dudit échantillon de salive stabilisée par la détection de séquences ARN ou ADN spécifiques d'origine humaine, bactérienne ou virale dans la dite fraction fluide.

**7.** La méthode selon la revendication 6, dans laquelle les séquences ADN ou ARN spécifiques sont choisies parmi :

i) Les séquences humaines sélectionnées parmi l'ARNm SSAT (SEQ ID n ° 62), l'ARNm H3F3A (SEQ ID n ° 63) et l'ARNm IL18 (SEQ ID n ° 64); et/ou
ii) les séquences bactériennes sélectionnées parmi *Capnocytophaga gingivalis* (ATCC 33624), *Prevotella melaninogenica* (ATCC 25845), *Streptococcus mitis* (ATCC 15914) et *Micrococcus luteus* (ATCC 53598D) ; et/ou
iii) les séquences virales du virus du papillome humain, de façon préférée ledit virus du papillome humain est le virus du papillome humain 16 (ATCC 45113) ou le virus du papillome humain 18 (ATCC 45152);

et de façon préférée, dans laquelle la détection d'au moins deux séquences d'ARNm telles que définies spécifiquement en i) et d'au moins une séquence bactérienne telle que spécifiquement définie en ii) dans la salive d'un sujet humain indique que ledit sujet humain présente un risque élevé de développer un cancer oral.

**8.** La méthode selon la revendication 7, dans laquelle la détection de l'ARNm humain de H3F3A (SEQ ID No 63)" de l'ARNm humain de SSAT (SEQ ID No 62) et du génome bactérien de *Streptococcus mitis* (ATCC 15914) dans la salive d'un sujet humain indique que le dit sujet humain présente un risque élevé de développer un cancer oral.

**9.** La méthode selon la revendication 7, dans laquelle :

i) La salive est stabilisée par l'utilisation d'une solution comprenant un sel tel que le thiocyanate de guanidinium, et/ou le sulfate d'ammonium, et/ou l'azoture de sodium, et optionnellement des inhibiteurs d'exo- et/ou d'endonucléases ; et/ou
ii) Les séquences d'ADN ou d'ARN spécifiques sont détectées en incubant l'ADN génomique ou l'ARN total avec une enzyme thermostable ayant une activité transcriptase inverse ARN-dépendante et une activité polymérase ADN-dépendante, de façon préférée la combinaison de RT-PCR et de PCR est réalisée dans un même tube à essais.

**10.** La méthode selon la revendication 7, dans laquelle la détection de la séquence de l'ARNm de H3F3A (SEQID No 63), de SSAT (SEQ ID No 62) ainsi que la détection des composés biochimiques organiques hexanenitrile, 2, 3-pentanedione, 3 methylthiophène et acétone au niveau de la salive d'un sujet humain indique que ledit sujet humain développe un cancer oral.

**11.** La méthode selon la revendication 1, dans laquelle :

i) La fraction volatile de salive est obtenue après avoir soumis un échantillon de salive à une température de 40°C et ce pendant au moins 10 minutes en utilisant la microextraction en phase solide (SPME) avec des fibres de CAR/PDMS ; et/ou
ii) La détection des composés biochimiques organiques de ladite fraction volatile est réalisée avec une chromatographie en phase gazeuse couplée à un spectromètre de masse.

**12.** La méthode selon la revendication 7, comprenant l'amplification et la détection d'au moins une séquence ADN ou ARN choisie dans le groupe comprenant : SEQ ID No 62 à 70.

**13.** La méthode selon la revendication 7, comprenant les étapes suivantes :

a) Recueillir un échantillon de salive dudit sujet humain dans un réceptacle stérile,
b) Stabiliser le dit échantillon en y ajoutant une solution comprenant un sel de guanidinium, comme du thiocyanate de guanidinium, et/ou du sulfate d'ammonium, et/ou de l'azoture de sodium, et éventuellement des inhibiteur d'exo- et/ou d'endo-nucléases,
c) L'extraction des acides nucléique s totaux d'origine bactérienne, virale, et humaine du précèdent échantillon stabilisé de salive humaine,
d) La précipitation et la purification des acides nucléiques totaux,
e) L'incubation des acides nucléiques totaux purifiés avec une enzyme thermostable ayant une activité de transcriptase inverse ARN-dépendante et ayant une activité polymérase ADN-dépendante et des amorces polynucléotidiques dans des conditions permettant à l'activité transcriptase inverse de ladite enzyme thermostable de synthétiser de l'ADNc à partir de l'ARN et l'amplification de l'ADN génomique et de l'ADNc à un niveau détectable par une réaction de polymérisation en chaîne,
f) La détection dans un test des séquences d'ADN amplifiées par l'hybridation avec une ou plusieurs sondes polynucléotidiques spécifiquse de :

i) séquences humaines sélectionnées parmi l'ARNm SSAT (SEQ ID n ° 62), l'ARNm H3F3A (SEQ ID n ° 63) et l' ARNm IL8 (SEQ ID n ° 64);ou
ii) séquences bactériennes sélectionnées parmi *Capnocytophaga gingivalis* (ATCC 33624, SEQ ID n ° 65), *Prevotella melaninogenica* (ATCC 25845, SEQ ID n ° 66), *Streptococcus mitis* (ATCC 15914, SEQ ID n ° 67) et *Micrococcus luteus* (ATCC 53598D, SEQ ID n ° 68), ou
iii) séquences virales sélectionnée parmi le virus du papillome humain 16 (ATCC 45 113, SEQ ID n ° 69) et le virus du papillome humain 18 (ATCC 45 152, SEQ ID N ° 70);

et, lesdites amorces et lesdites sondes utilisées pour l'amplification et la détection comprenant au moins une séquence i), ii) ou iii) sont de façon préférées sélectionnées dans le groupe consistant en SEQ ID No 1 à SEQ ID No 60.

**14.** La méthode selon la revendication 1, comprenant les étapes suivantes :

a) Recueillir un échantillon de salive dudit sujet humain dans un réceptacle stérile,
b) Stabiliser le dit échantillon en y ajoutant une solution comprenant un sel de guanidinium, comme du thiocyanate de guanidinium, et/ou du sulfate d'ammonium, et/ou de l'azoture de sodium,
c) Extraire la fraction volatile dudit échantillon stabilisé en le chauffant à une température de 40° pendant au moins 10 minutes en utilisant par exemple la microextraction en phase solide (SPME) afin de séparer la fraction volatile,
d) La détection d'au moins un composé biochimique organique en utilisant à titre d'exemple une chromatographie en phase gazeuse associée a un spectromètre de masse, le dit au moins un composé biochimique organique est et de façon préférée choisi parmi : la 3- pentanedione (numéro CAS 600- 14- 6), le 3- méthylthiophène (numéro CAS 616- 44- 4), l'acétone (numéro CAS 67- 64- 1), hexanenitrile (numéro CAS 628- 73- 9), le benzaldéhyde (numéro CAS 100- 52- 7), la 3- méthyl- 2- pentanone (numéro CAS 565- 61- 7 ), le 2, 3- butanedione (numéro CAS 431- 03- 8 numéro), 2- propanol (numéro CAS 67- 63- 0), l'acétate d'éthyle (numéro CAS 141- 78- 6), le 1- propanol (numéro CAS 71- 23- 8), l'hexanal (numéro CAS 66- 25- 1), la 5- méthyl- 3- Hexène- 2- one (numéro CAS 5166- 53- 0), le m- xylène (numéro CAS 108- 38- 3), le p- xylène (numéro CAS 106- 42- 3), le 2- méthyl- 2- butenal (E) (numéro CAS 497- 03- 0), le phénol (numéro CAS 108- 95- 2), le butanal (numéro CAS 123- 72- 8), la méthylbutanone (numéro CAS: 563- 80- 4), le 2- méthyl- 2- butène (numéro CAS 513- 35- 9), le 2- méthyl- 1- ène (numéro CAS 115- 11- 7) et le (cis) 1, 2 diméthyl- cyclopropane (numéro CAS: 930- 18- 7) .

**15.** Une utilisation pour la mise en oeuvre d'une méthode selon la revendication 14, d'un kit comprenant :

a) Un dispositif stérile pour la collecte d'un échantillon de salive, pouvant contenir optionnellement un réactif de collecte,
b) Un agent conservateur,
c) Au moins un capteur électronique,
d) Eventuellement, un marqueur moléculaire de contrôle.

Figure 1

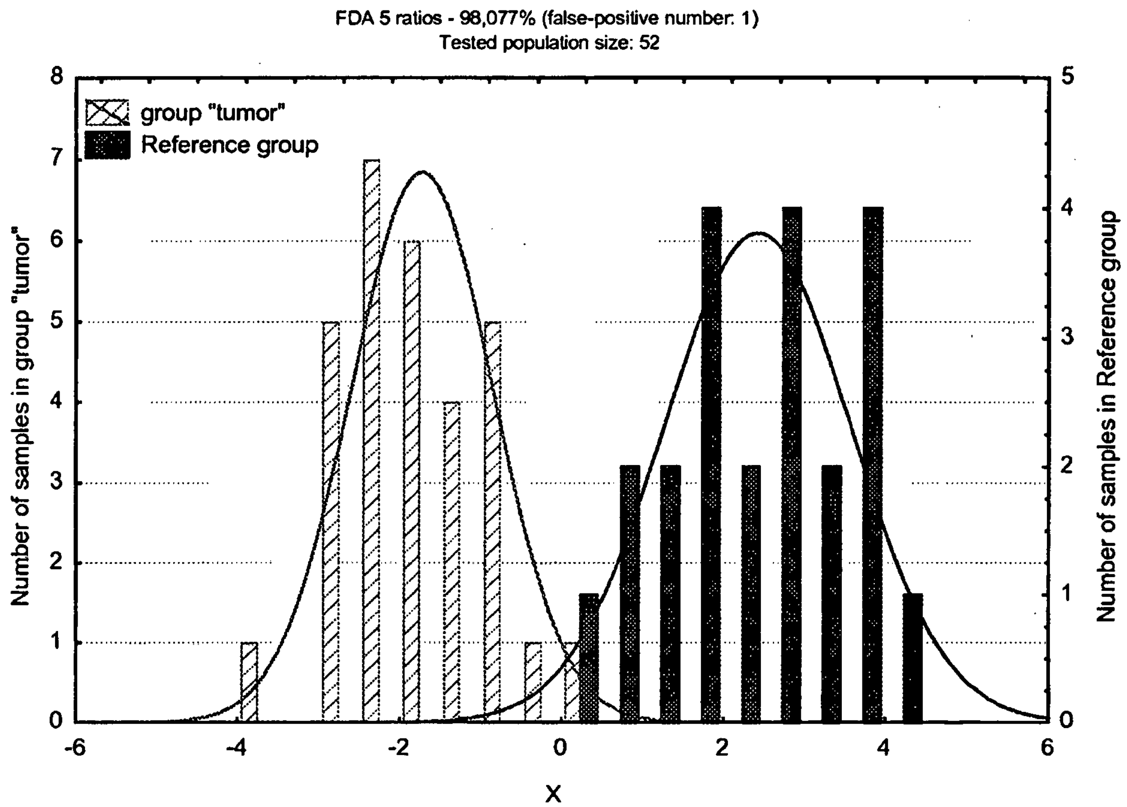
FDA 5 ratios - 98,077% (false-positive number: 1)
Tested population size: 52
group "tumor"
Reference group
Number of samples in group "tumor"
Number of samples in Reference group
X
8
7
6
5
4
3
2
1
0
-6
-4
-2
0
2
4
6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61086019 A
- WO 2006020005 A
- WO 2005081867 A
- WO 9109944 A
- US 61086019 B


### Non-patent literature cited in the description


- **EPSTEIN, J.B. et al.** *J Can Dent Assoc,* 2002, vol. 68, 617-621
- **MAO, L. et al.** *Cancer Cell,* 2004, vol. 5, 311-316
- **REHAK, N.N. et al.** *Clin Chem Lab Med,* 2000, vol. 38, 335-343
- **WONG DT.** *American Scientist,* 2008, vol. 96
- **STAMEY, F.R. et al.** *J Virol Methods,* 2003, vol. 108, 189-193
- **MERCER, D.K. et al.** *FEMS Microbiol Lett,* 2001, vol. 200, 163-167
- **ZIMMERMANN BG et al.** *Oral Oncology,* 2008, vol. 44, 425-429
- **STRECKFUS, CF. ; BIGLER, L.R.** *Oral Dis,* 2002, vol. 8, 69-76
- **LAWRENCE H.P. et al.** *J Can Dent Assoc,* 2002, vol. 68, 170-174
- **MAGER D.L. et al.** *J Transl Med.,* 2005, vol. 3, 27
- **HOOPER J.S et al.** *Journal of Clinical Microbiology,* May 2006, 1719-1725
- **LI et al.** *Journal of Applied Microbiology,* 2004, vol. 97, 1311-1318
- **RAGHUNAD N. et al.** *J. Radiol.,* 2003, vol. 76, S11-S22
- **TAKEUCHI T. et al.** *FEMS Microbiol Lett.,* 01 November 2000, vol. 192 (l), 133-8
- **D'SOUZA et al.** *N Engl J Med.,* 10 May 2007, vol. 356 (19), 1944-56
- **HERRERO R et al.** *J Natl Cancer Inst.,* 03 December 2003, vol. 95 (23), 1772-83
- **ROSENQUIST K. et al.** *Acta Otolaryngol.,* September 2007, vol. 127 (9), 980-7
- **SIDRANSKY, D.** *Nat Rev Cancer,* 2002, vol. 2, 210-219
- **HOLLSTEIN, M. et al.** *Science,* 1991, vol. 253, 49-53
- **LIU, T. et al.** *Genes Chromosomes Cancer,* 2000, vol. 27, 17-25
- **GRODEN, J. et al.** *Cell,* 1991, vol. 66, 589-600
- **LIAO P.H. et al.** *Oral Oncol,* 2000, vol. 36, 272-276
- **EL-NAGGAR, A.K. et al.** *JMolDiagn,* 2001, vol. 3, 164-170
- **YANG LI et al.** *Journal of Clinical Oncology,* 2006, vol. 24 (11), 1754-1760
- **HU S.** *J Dent Res.,* December 2006, vol. 85 (12), 1129-33
- **MASHIR A.** *Advanced Powder Technology,* 2009
- **VOLOZHIN et al.** *Stomatologiia (mosk,* 2001, vol. 80 (1), 9-12
- **BAHAR et al.** Salivary analysis in oral cancer patients - DNA and protein oxidation, reactive nitrogen species, and antioxidant profile. *CANCER,* January 2007, vol. 109 (1), 54-59
- **PAULING L. et al.** *Proc.Natl.Acad.Sci.USA,* 1971, vol. 68, 2374-2376
- **CHAN H.P. et al.** *Lung Cancer,* 2009
- **SONG G et al.** Quantitative breath analysis of volatile organic compounds of lung cancer patients. *Lung Cancer,* 2009
- **PARK NJ.** *Clin Chem,* 2006, vol. 52, 2303-4
- **JIANG J et al.** *Archives of Oral Biology,* 2008
- **MILLS G et al.** *Journal of Chromatography,* 2000
- **SONG C.** *Lung Cancer,* 2009
- **GARCIA-ESTEBAN M et al.** *Talanta,* 2004
- **CHO S.M.** *Sensors and Actuators,* 2006